# EUROPÄISCHE PATENTSCHRIFT

(11) **EP 3 720 943 B1**
(45) Veröffentlichungstag und Bekanntmachung des Hinweises auf die Patenterteilung: **02.12.2026**
(21) Anmeldenummer: 18793622.4
(22) Anmeldetag: 18.10.2018
(51) Int. Cl.: C12M 1/26, C12M 1/12, C12M 1/34

(54) **VORRICHTUNG UND VERFAHREN ZUR UNTERSUCHUNG EINES MEDIUMS**
DEVICE AND METHOD FOR EXAMINING A MEDIUM
DISPOSITIF ET PROCÉDÉ D'ANALYSE D'UN MILIEU

(30) Priorität: 06.12.2017 DE 102017011263
(43) Veröffentlichungstag der Anmeldung: 14.10.2020
(73) Patentinhaber: Sartorius Stedim Biotech GmbH, 37079 Göttingen (DE)
(72) Erfinder: GRIMM, Christian, 37308 Heilbad Heiligenstadt (DE); WEICHERT, Henry, 04720 Westewitz (DE); BECKER, Mario, Brooklyn New York 11222 (US)
(74) Vertreter: Novagraaf International SA
(86) Internationale Anmeldenummer: PCT/EP2018/078624
(87) Internationale Veröffentlichungsnummer: WO 2019/110185

(56) Entgegenhaltungen:
- EP-A1- 1 922 987
- EP-B1- 2 405 802
- WO-A2-2009/120269
- DE-A1- 102006 019 242
- DE-A1- 102012 003 113
- US-A- 4 700 560
- US-A- 5 800 692
- US-A1- 2016 201 152

## Beschreibung

Die Erfindung betrifft eine Vorrichtung und ein Verfahren zur Untersuchung eines Mediums im Inneren eines Bioreaktors, insbesondere eines Einwegbioreaktors.

Der Trend der biopharmazeutischen Industrie geht immer stärker zu sogenannten Einwegbioreaktoren wie z.B. Beutel aus Kunststofffolie, welche auch Bags genannt werden. Die Erwartungshaltung von Endanwendern dieser Einwegbioreaktoren ist es, die gleichen Systeme, Messmethoden und Parameter zur Verfügung zu haben wie in klassischen Edelstahlbioreaktoren. Insbesondere sollen verwendete Messmethoden in Bezug auf ihre Genauigkeit, ihre Zuverlässigkeit und den zu untersuchenden Messbereich die gleichen Anforderungen erfüllen wie bei Edelstahlbioreaktoren. Dazu werden bereits heute verschiedene Sensoren, wie etwa optische pH-Sensoren und optische Sensoren für gelösten Sauerstoff in die Einwegbioreaktoren integriert. Dies geschieht u.a. durch Einschweißen spezieller Ports und spezieller Haltevorrichtungen in die Reaktorwand des Einwegbioreaktors.

Eine Messung von Nährstoffen, wie etwa Glucose oder Glutamin bzw. Metaboliten wie etwa Lactat oder Glutamat sind derzeit nur über eine manuelle Probennahme und anschließende Laboranalyse oder eine Tauchsonde aus Edelstahl möglich. Die Tauchsonden sind dabei so konzipiert, dass sie durch eine Membran, per Dialyse oder Filtration, eine kontinuierliche, repräsentative Menge Medium aus dem Reaktor entnehmen und einem Analysesystem zuführen können.

Eine Messung eines Zielproteins, z.B. eines monoklonalen Antikörpers ist sogar nur über eine Probennahme, zusätzliche Aufreinigung und anschließende Laboranalyse (HPLC oder ELISA Techniken) möglich.

Die allgemein übliche Methode zur Sterilisierung von Einwegbioreaktoren ist die Bestrahlung mit bis zu 50 kGy Gammastrahlung. Um Einwegbioreaktoren während der Probennahme steril zu halten, ist es erforderlich, dass ein Einführen einer Tauchsonde zur kontinuierlichen zellfreien Probennahme nur über einen speziellen Port unter besonders sensibler Beachtung von Hygienebedingungen möglich ist. Dennoch kann es durch Verwendung einer solchen Sonde zu ungewollter Kontamination des Zellkulturprozesses oder zur Beschädigung des Einwegbioreaktors kommen.

Derzeit verwendete Einwegbioreaktoren sind im Wesentlichen in drei Haupttypen verfügbar. Gerührte Systeme mit Impeller und Sparger zur Begasung und Agitation, geschüttelte Systeme und Systeme, die Belüftung und Mischung über eine Wellenbewegung realisieren, welche auch mit RM für "rocking motion" bezeichnet werden. Insbesondere in derzeitigen RM-Systemen ist ein Einbringen von Edelstahltauchsonden auf Grund der Bauform des Einwegbioreaktors und der Bewegung, die eine permanente Flüssigkeitsüberdeckung der Tauchsonden verhindert, nicht ohne weiteres möglich.

Das Dokument EP 1 922 987 A1 offenbart eine Probenahmevorrichtung zum Gewinnen einer Probe eines Analyten mit einer Zuleitung und einer Ableitung sowie einer Analytaufgabekammer in fluidleitender Verbindung zwischen der Zuleitung und der Ableitung. Die Analytaufgabekammer besitzt eine Öffnung, die mit einer analytdurchlässigen Membran zum Durchtretenlassen des Analyten aus einem Bereich außerhalb der Analytaufgabekammer in die Analytaufgabekammer versehen ist. Die Fläche der Öffnung der Analytaufgabekammer beträgt höchstens das 400fache der Mindest-Querschnittsfläche der Ableitung. Mit dieser Vorrichtung soll eine Totzeit zwischen einem Durchtreten des Analyten durch die Membran und dem Nachweis am Sensor verringert werden.

Das Dokument US 5,800,692 offenbart einen Vortrennprozessor zur Verwendung bei einer Kapillarelektrophorese. Der Vortrennprozessor enthält ein Probenverarbeitungsmaterial zum Aufkonzentrieren oder chemischen Bearbeiten einer Probe, oder zum Katalysieren einer chemischen Reaktion. Es eignet sich besonders zum Aufkonzentrieren verdünnter Proben oder zur Reinigung kontaminierter Proben.

Das Dokument US 2016/0201152 beschreibt die Verarbeitung von Biomasse-Rohstoffen wie z. B. Pflanzenbiomasse, Tierbiomasse und Biomasse aus Siedlungsabfällen, um nützliche Produkte wie Kraftstoffe herzustellen. Beispielsweise werden Systeme beschrieben, die zum Trennen von Feststoffen und hochmolekularen Spezies von Flüssigkeiten aus biologisch verarbeiteten Biomasse-Materialschlämmen nützlich sein können.

Der Erfindung liegt die Aufgabe zugrunde, die Untersuchung und/oder Aufreinigung eines Mediums im Inneren eines Bioreaktors zu verbessern.

Diese Aufgabe wird durch die Gegenstände der unabhängigen Ansprüche gelöst. Bevorzugte Ausführungsformen sind die Gegenstände der abhängigen Ansprüche.

Ein Aspekt betrifft eine Vorrichtung zur Untersuchung eines Mediums im Inneren eines Bioreaktors, insbesondere eines Einwegbioreaktors, mit einem Probenentnahmemodul zur Entnahme einer Probe des Mediums. Dabei weist das Probenentnahmemodul einen Entnahmebereich auf, der in Berührkontakt mit dem Medium im Inneren des Bioreaktors anordenbar ist. Am Entnahmebereich des Probenentnahmemoduls sind zumindest zwei unterschiedliche Membranen zur Entnahme einer Probe des Mediums angeordnet.

Die Vorrichtung ist dazu ausgebildet und vorgesehen, das im Inneren eines Bioreaktors angeordnete Medium online zu untersuchen, d.h. im Wesentlichen ohne den biologischen Prozess im Inneren des Bioreaktors zu stören und/oder zu unterbrechen. Auch wenn die Vorrichtung zur Untersuchung Inhalts eines Einwegbioreaktors ausgebildet ist, kann die Vorrichtung auch dazu ausgebildet sein, den Inhalt eines Edelstahlbioreaktors und/oder eines Pallettanks zu untersuchen.

Das Probenentnahmemodul kann als eine Tauchsonde ausgebildet sein und/oder eine Tauchsonde aufweisen, insbesondere eine aus Edelstahl ausgebildete Tauchsonde. Das Probenentnahmemodul kann auch einen Einschweißport aufweisen und/oder als ein Einschweißport ausgebildet sein, in welchem der oder die Membran(en) angeordnet ist bzw. sind. Der Einschweißport kann hierbei flächig ausgebildet sein, also z.B. eine dem Inneren des Bioreaktors zugewandte Bioreaktorfläche und/oder eine dem Inneren des Bioreaktors abgewandten Außenfläche aufweisen. Zuleitungen zu dem Einschweißport können an einer dem Inneren des Bioreaktors abgewandten Seite des Probenentnahmemoduls angeordnet sein, z.B. an der voranstehend erwähnten Außenfläche des Einschweißports. Das Probenentnahmemodul kann auch einen Schlauch aufweisen und/oder im Wesentlichen schlauchförmig ausgebildet sein. Hierbei kann zumindest eine der Membranen als ein Teil der Schlauchwand ausgebildet sein. Weiterhin kann das Probenentnahmemodul auch eine Röhre aufweisen und/oder im Wesentlichen rohrförmig ausgebildet sein

Das Probenentnahmemodul kann auch zur gezielten Aufreinigung des Mediums des Bioreaktors verwendet werden.

Der Entnahmebereich des Probenentnahmemoduls kann dazu ausgebildet und vorgesehen sein, in einer Betriebsposition das Innere des Bioreaktors eingeführt zu werden. In der Betriebsposition der Vorrichtung ist der Entnahmebereich in Berührkontakt mit dem (z.B. biologischen) Medium im Inneren des Bioreaktors stehend angeordnet.

Der Entnahmebereich kann z.B. als ein Sondenteil ausgebildet sein, der z.B. eine Sondenspitze enthält. Der Entnahmebereich kann eine Sondenspitze aufweisen.

Der Entnahmebereich kann in der Betriebsposition z.B. von außen durch einen Port in der Reaktorwand des Bioreaktors hindurch in das Innere des Bioreaktors geführt sein.

Das Probenentnahmemodul kann einen externen Modulbereich aufweisen, der in der Betriebsposition außerhalb des Bioreaktors angeordnet ist. Der Entnahmebereich kann mit dem externen Modulbereich über zumindest eine Transportleitung durch das Innere des Probenentnahmemoduls verbunden sein, in welcher eine Probe des Mediums aus dem Bioreaktor heraus transportiert werden kann.

Das Probenentnahmemodul kann dazu ausgebildet sein, an einer Halterung so am und/oder relativ zum Bioreaktor befestigt zu werden, dass der Entnahmebereich durch die Reaktorwand hindurch ins Innere des Bioreaktors ragt, während der externe Modulbereich des Probenentnahmemoduls außerhalb des Bioreaktors angeordnet ist.

Der Entnahmebereich kann einen hohlen Innenbereich aufweisen, in welchen die Probe(n) des Mediums eingebracht werden kann. Der hohle Innenbereich kann als Teil der Transportleitung ausgebildet sein.

Das Probenentnahmemodul dient zur Entnahme einer Probe des Mediums aus dem Inneren des Bioreaktors. Hierbei kann eine dialytische Probennahme erfolgen, wobei eine Probe entnommen wird, ohne das Gesamtvolumen zu reduzieren. Das Medium ist hierbei üblicherweise ein Gemisch aus unterschiedlichen Bestandteilen wie z.B. Zellen, Proteinen, Antikörper und/oder .kleineren Molekülen wie z.B. Glucose. Das Entnehmen einer Probe bedeutet hierbei, dass eine Probe zumindest eines dieser Bestandteile des Mediums entnommen wird, nicht notwendigerweise ein Bruchteil aller unterschiedlichen Bestandteile des Mediums. Hierbei können schlauchförmige Reinigungsmodule verwendet werden, z.B. Filervorrichtungen.

Die entnommene Probe des Mediums kann nach dem Entnehmen untersucht werden, z.B. nachdem sie zunächst zum externen Modulbereich des Probenentnahmemoduls transportiert wurde und von dort weiter zu einem Analysemodul, welches die eigentliche Analyse vornimmt. Das Probenentnahmemodul kann z.B. an ein externes Analysesystem angeschlossen und/oder gekoppelt sein, z.B. an einen Massenspektrometer, einen Gas-Chromatographen, ein Flüssig-Chromatographie-System wie ein HPLC/UPLC-System (was für High Performance Liquid Chromatography bzw. Ultra Performance Liquid Chromatography steht) und/oder ein enzymatisches Analysesystem wie Trace.

Am Entnahmebereich des Probenentnahmemoduls sind zumindest zwei unterschiedliche Membranen zur Entnahme einer Probe des Mediums angeordnet. Die beiden Membranen können sich z.B. darin unterscheiden, welche Bestandteile des Mediums sie jeweils als Probe entnehmen können. Die Membranen können sich insbesondere in ihrer Cutoff-Porengröße unterscheiden und/oder unterschiedlich durchlässig und/oder unterschiedlich stark ausgebildet sein. Das Probenentnahmemodul kann insbesondere genau zwei unterschiedliche Membranen zur Probeentnahme aufweisen. Alternativ kann das Probenentnahmemodul auch mehr als zwei unterschiedliche Membranen aufweisen.

Wie eingangs erläutert, gibt es bislang lediglich Tauchsonden zur Bestimmung der Konzentration von Nährstoffen. Diese weisen eine bestimmte Membran auf, die durchlässig für Nährstoffe ist.

Die Erfindung ist bei der Untersuchung des Mediums nicht auf diesen einen Bestandteil des Medium beschränkt, wie bei einer herkömmlichen Tauchsonde, sondern kann die Untersuchung des Mediums auf mehrere unterschiedliche Bestandteile erweitern. Dies ermöglicht die Verwendung zweier unterschiedlicher Membranen zur Probennahme, wobei sich die zumindest zwei Membranen zumindest dadurch unterscheiden, dass sie unterschiedlich große Cutoff-Porengrößen aufweisen.

Dadurch wird eine neue Möglichkeit bereitgestellt, eine z.B. zellfreie Probenmenge kontinuierlich aus einem Einwegbioreaktor zu entnehmen und einem Analysator zuzuführen. Diese ist dann z.B. auch auf RM-Systeme anwendbar, also Bioreaktoren, bei denen die Einwegbioreaktoren über eine Schaukelbewegung durchmischt werden.

Die Vorrichtung kann auch zur Aufreinigung verwendet werden, also z.B. als ein schlauchförmiges Reinigungsmodul ausgebildet sein.

Mit der Vorrichtung ist es möglich, in einem Einwegbioreaktor online die Konzentration an Nährstoffen und Metaboliten (wie z.B. Glukose, Laktat, Glutamin, Glutamat, biogene Aminosäuren, Glyzerol, Azetat, Ethanol oder Methanol) sowie an Zielproteinen (wie z.B. monoklonale Antikörper, Hormone, Wachstumsfaktoren, Interleukine, Interferone) gleichzeitig zu bestimmen.

In einer Ausführungsform weist die Vorrichtung lediglich eine einzige Membran auf, mittels der Proben entnommen werden können. Hierbei kann die Membran derart ausgebildet sein, dass dem Medium z.B. Proben mit zumindest zwei unterschiedlichen Teilchendurchmessern entnommen werden können.

Gemäß einer Ausführungsform ist jede der zumindest zwei Membranen an einem Außenbereich des Entnahmebereichs angeordnet, so dass jede der zumindest zwei Membranen jeweils in (unmittelbarem) Berührkontakt mit dem Medium im Inneren des Bioreaktors anordenbar ist. Hierbei können die beiden Membranen insbesondere gleichzeitig in Berührkontakt mit dem Medium stehen, nämlich in der Betriebsposition der Vorrichtung. Mit anderen Worten ist der Entnahmebereich an zwei unterschiedlichen Stellen seines Außenbereichs, welcher sich in Berührkontakt mit dem Medium befindet, von jeweils einer der beiden Membranen begrenzt. Hierbei ist jede der Membranen so angeordnet, dass sie jeweils einen Übertritt zumindest eines Bestandteils des Mediums durch die Membran hindurch ins Innere des Probenentnahmemoduls zulässt; z.B. in jeweils eine Transportleitung des Probenentnahmemoduls.

Gemäß einer Ausführungsform unterscheiden sich die zumindest zwei Membranen zumindest dadurch, dass sie unterschiedlich große Cutoff-Porengrößen aufweisen. Die Cutoff-Porengröße kann z.B. in kDa (Kilodalton) angegeben werden und bestimmt eine spezifische Teilchengröße. Bestandteile des Medium mit einer spezifischen Teilchengröße, die kleiner ist als die durch die Cutoff-Porengröße bestimmte spezifische Teilchengröße, können durch die jeweilige Membran hindurch diffundieren. Weicht die Teilchengröße der Bestandteile zu stark nach oben von der Cutoff-Porengröße ab, so können die Teilchen nicht oder nur sehr beschränkt durch die Membran hindurch diffundieren.

Gemäß einer Weiterbildung dieser Ausführungsform weist eine erste Membran der zumindest zwei Membranen eine Cuttoff-Porengröße von 50 kDa bis 200 kDa und eine zweite Membran der zumindest zwei Membranen eine Cuttoff-Porengröße von 10 kDa bis 30 kDa auf. Hierbei eignet sich die zweite Membran mit der kleineren Cutoff-Porengröße insbesondere zum Bestimmen der Konzentration von kleineren Molekülen wie Nährstoffen (z.B. Glucose oder Glutamin) und Metaboliten (z.B. Lactat oder Glutamat). Die erste Membran mit der größeren Cutoff-Porengröße eignet sich hierbei insbesondere zum Bestimmen der Konzentration von größeren Molekülen wie zumindest eines vorbestimmten Zielproteins (z.B. eines Antikörpers).

Gemäß einer Ausführungsform ist eine erste Membran der zumindest zwei Membranen dazu ausgebildet und angeordnet, in Berührkontakt mit dem Medium einen Übertritt von Proteinen aus dem Medium in das Probenentnahmemodul zuzulassen. Eine zweite Membran der zumindest zwei Membranen ist dazu ausgebildet und angeordnet, in Berührkontakt mit dem Medium einen Übertritt von Nährstoffen und/oder Metaboliten aus dem Medium in das Probenentnahmemodul zuzulassen. Hierbei kann es sich also z.B. um Membranen mit den voranstehend genannten Cutoff-Porengrößen handeln. Bei Verwendung dieser Membranen können mit einer Vorrichtung zwei unterschiedliche Bestandteile des Medium ermittelt und/oder untersucht werden.

Gemäß einer Ausführungsform weist das Probenentnahmemodul zumindest eine Transportleitung zum Transportieren eines Adsorbers durch den Entnahmebereich entlang der Transportleitung auf. Hierbei begrenzt jede der zumindest zwei Membranen die Transportleitung nach außen. Mit anderen Worten ist jede der beiden Membranen zwischen der Transportleitungen und einem Außenbereich des Entnahmebereich angeordnet. In diesem Außenbereich ist in der Betriebsposition auch das zu untersuchende Medium angeordnet. Die Begrenzung nach Außen hin ermöglicht somit den unmittelbaren Berührkontakt der Membranen mit dem Medium. Die Transportleitung ist als Hohlraum ausgebildet, in welchem der Adsorber geführt ist. Der Adsorber dient zur Aufnahme der Proben, die in der Betriebsposition durch die jeweilige Membran hindurch in die Transportleitung an den Adsorber diffundieren können. Hierbei können unterschiedliche Adsorber für die unterschiedlichen Bestandteile vorgesehen sein. So können durch die eine oder mehreren Transportleitungen unterschiedliche Adsorber transportiert werden, die jeweils als Adsorber für die jeweils zu nehmende Probe ausgebildet sind.

Gemäß einer Ausführungsform werden zumindest zwei unterschiedliche Adsorber durch die Transportleitung(en) geführt, welche jeweils als Adsorber für die unterschiedlichen, zu untersuchenden Bestandteile des Mediums ausgebildet sind. So wird eine Diffusion der unterschiedlichen Bestandteile des Mediums durch die unterschiedlichen Membranen mit einem jeweils dafür spezifischen Adsorber ausgelöst und/oder unterstützt.

Gemäß einer Weiterbildung dieser Ausführungsform weist die Vorrichtung ein Steuermodul zum Steuern eines Adsorberflusses durch die zumindest eine Transportleitung des Probenentnahmemoduls auf. Das Steuermodul kann einen Prozessor aufweisen und/oder als Computer ausgebildet sein. Das Steuermodul kann insbesondere ein oder mehrere Ventile und/oder Pumpen so ansteuern, dass der oder die Adsorber mit einer vorbestimmbaren und/oder veränderlichen Geschwindigkeit durch die Transportleitung geführt wird. Hierbei kann/können der/die Adsorber insbesondere für eine ansteuerbare und/oder vorbestimmbare Zeit angehalten werden, um eine Anreicherung mit den zu untersuchenden Bestandteilen des Mediums zu ermöglichen. Anschließend kann/können der/die mit der oder den Probe(n) angereicherte(n) Adsorber weitergeführt werden, insbesondere aus dem Entnahmebereich und dem Probenentnahmemodul heraus zu einem Analysesystem oder einem Analysemodul.

Gemäß einer zusätzlichen oder alternativen Weiterbildung der Ausführungsform weist das Probenentnahmemodul genau eine Transportleitung durch den Entnahmebereich auf und die zumindest zwei Membranen sind in Transportrichtung durch die Transportleitung hintereinander angeordnet. Hierbei werden zwei zueinander in Transportrichtung versetzte Abschnitte der Transportleitung mit den jeweils zu untersuchenden Bestandteilen des Mediums angereichert, bevor der oder die so angereicherte(n) Adsorber aus dem Probenentnahmemodul herausgeführt wird/werden. Somit wird nur die Ausbildung einer einzigen Transportleitung im Entnahmebereich benötigt, was eine relativ einfache und somit kostengünstige Herstellung des Probenentnahmemoduls ermöglicht.

Alternativ hierzu weist das Probenentnahmemodul zumindest zwei Transportleitungen durch den Entnahmebereich auf und die zumindest zwei Membranen sind in unterschiedlichen Transportleitungen angeordnet. Hierbei kann für jeden zu untersuchenden Bestandteil, und somit für jede unterschiedliche Membran, eine eigene Transportleitung vorgesehen und ausgebildet sein. Hierbei kann die Ansteuerung des oder der Adsorbers einfacher ausgebildet sein, da in jeder Transportleitung nur eine Anreicherung erfolgen muss. Weiterhin muss durch jede Transportleitung nur jeweils eine Adsorberart transportiert werden. Weiterhin können die einzelnen Bestandteile des Mediums getrennt voneinander untersucht werden, z.B. in unterschiedlichen Analysesystemen und/oder unterschiedlichen Bestandteilen eines Analysemoduls.

Gemäß einer Ausführungsform sind die Membranen als Dialysemembranen und/oder semipermeable Membranen ausgebildet. Solche Membranen eignen sich besonders für Bioreaktoren, da die Gefahr einer Verunreinigung des Inhalts des Bioreaktors durch die Vorrichtung reduzieren.

Gemäß einer Ausführungsform weist die Vorrichtung ein Analysemodul zur Untersuchung einer von dem Probenentnahmemodul entnommenen Probe des Mediums auf. Das Analysemodul ist hierbei als Bestandteil der Vorrichtung ausgebildet. Das Analysemodul kann mehrteilig ausgebildet sein, insbesondere mit zumindest zwei unterschiedlichen Messzellen zur Untersuchung zumindest zweier unterschiedlicher Bestandteile des Mediums. Die zumindest eine Probe kann dem Analysemodul z.B. über eine Transportleitung zugeführt werden, die durch das Probenentnahmemodul hindurch verlaufend angeordnet ist.

In einer Weiterbildung dieser Ausführungsform ist das Analysemodul dazu ausgebildet, durch Untersuchung der entnommenen Probe des Mediums eine Konzentration zumindest zweier verschiedener Bestandteile im Medium zu bestimmen. Hierbei können insbesondere die beiden Konzentrationen der zwei unterschiedlichen Bestandteile bestimmt werden, die durch die beiden unterschiedlichen Membranen diffundiert sind. Die Vorrichtung kann hierbei zur online Bestimmung der Konzentration zweier unterschiedlicher Bestandteile des Mediums ausgebildet sein. Die unterschiedlichen Bestandteile des Mediums können sich z.B. durch ihre mittlere Molekülgröße unterscheiden.

Ein Aspekt betrifft ein Verfahren zur Untersuchung eines Mediums im Inneren eines Bioreaktors, insbesondere eines Einwegbioreaktors, mit den Schritten:
- Anordnen eines Entnahmebereichs eines Probenentnahmemoduls in Berührkontakt mit dem Medium im Inneren des Bioreaktors;
- Entnehmen zumindest einer Probe des Mediums durch zwei unterschiedliche Membranen des Entnahmeteils hindurch;
- Untersuchen der entnommenen Probe des Mediums.

Das Verfahren kann insbesondere mit einer Vorrichtung gemäß dem voranstehend beschriebenen Aspekt durchgeführt werden. Deswegen betreffen die zu der Vorrichtung gemachten Ausführungen auch das Verfahren und umgekehrt.

Insbesondere bei der Untersuchung von Antikörpern oder Zielproteinen mit bestimmten Techniken wie MS oder HPLC kann es sinnvoll sein, die Konzentration der Antikörper noch weiter zu erhöhen. Die Konzentration im Absorber könnte zu gering sein. Deswegen kann das Verfahren z.B. durch eine nachgeschaltete Chromatographie ergänzt werden, insbesondere in Form eines Capture steps (z.B. Protein A Bind + Elute).

Mit dem Verfahren können z.B. zumindest zwei Proben zumindest zweier unterschiedlicher Bestandteile des Mediums genommen werden. Alternativ kann lediglich eine einzige Probe genommen werden, die zeitlich und/oder räumlich hintereinander an jeder einzelnen der zumindest zwei Membranen genommen wird. Mit anderen Worten kann zunächst eine Probe eines ersten Bestandteiles an einer ersten Membran genommen werden, anschließend diese Probe ergänzt werden an einer zwei (dazu unterschiedlichen) Membran.

Hierbei können die unterschiedlichen Bestandteile des Mediums durch jeweils eine der beiden unterschiedlichen Membranen hindurch in das Probenentnahmemodul diffundieren.

Das Verfahren kann online durchgeführt werden und (z.B. in regelmäßigen Abständen) wiederholt werden. Mit dem Verfahren kann somit z.B. die Konzentration von zumindest zwei unterschiedlichen Bestandteilen des Mediums im Bioreaktor untersucht, bestimmt und/oder überwacht werden.

In einer Ausführungsform werden die beiden Membranen so in Berührkontakt mit dem Medium angeordnet, dass Bestandteile des Mediums durch jede der beiden unterschiedlichen Membranen hindurch in zumindest eine Transportleitung durch den Entnahmebereich des Probenentnahmemoduls übertreten. Die zumindest eine Transportleitung verläuft zumindest durch den Entnahmebereich des Probenentnahmemoduls hindurch und dient zur Aufnahme und/oder zum Transport der zumindest einen Probe.

In einer Weiterbildung der Ausführungsform wird ein Adsorberfluss durch die zumindest eine Transportleitung so angesteuert, dass die Bestandteile des Mediums durch jede der Membranen für einen vorbestimmbaren Zeitraum in einen in der Transportleitung ruhenden Adsorber übertreten, bevor der angereicherte Adsorber entlang der zumindest einen Transportleitung zu einem Analysemodul transportiert wird.

In einer Ausführungsform weist die Probe des Mediums zumindest zwei Bestandteile aufweist, welche durch eine Luftblase getrennt voneinander durch die beiden unterschiedlichen Membranen hindurch entnommen werden. Allgemein können die Bestandteile der Probe räumlich und/oder zeitlich getrennt voneinander durch die beiden Membranen hindurch entnommen werden. Ein erster Bestandteil der Probe, der durch die erste Membran hindurch genommen wird, kann auch als eine erste Probe und/oder erste Fraktion bezeichnet werden und z.B. zumindest eine erste Art Probenteilchen aufweisen. Ein zweiter Bestandteil der Probe, der durch die zweite Membran hindurch genommen wird, kann auch als eine zweite Probe und/oder zweite Fraktion bezeichnet werden und z.B. zumindest eine zweite Art Probenteilchen aufweisen. Die beiden Fraktionen werden zumindest räumlich getrennt voneinander genommen. Die beiden Fraktionen können z.B. durch eine Luftblase voneinander getrennt werden. Die Luftblase kann durch eine gezielte Ansteuerung der in den Transportleitungen enthaltenen Fluide erzeugt werden.

Ein Aspekt betrifft die Verwendung einer Vorrichtung zur Untersuchung eines Mediums im Inneren eines Bioreaktors gemäß dem voranstehend beschriebenen Aspekt zum Durchführen des Verfahrens zur Untersuchung eines Mediums im Inneren eines Bioreaktors gemäß dem weiteren voranstehend beschriebenen Aspekt.

Im Rahmen dieser Erfindung können die Begriffe "im Wesentlichen" und/oder "etwa" so verwendet sein, dass sie eine Abweichung von bis zu 5% von einem auf den Begriff folgenden Zahlenwert beinhalten; eine Abweichung von bis zu 5° von einer auf den Begriff folgenden Richtung und/oder von einem auf den Begriff folgenden Winkel.

Begriffe wie oben, unten, oberhalb, unterhalb, usw. beziehen sich - sofern nicht anders spezifiziert - auf das Bezugssystem der Erde in einer Betriebsposition des Gegenstands der Erfindung.

Die Erfindung wird nachfolgend anhand von in Figuren gezeigten Ausführungsbeispielen näher beschrieben. Hierbei können gleiche oder ähnliche Bezugszeichen gleiche oder ähnliche Merkmale der Ausführungsformen kennzeichnen. Einzelne in den Figuren gezeigte Merkmale können in anderen Ausführungsbeispielen implementiert sein. Es zeigen:
- Figur 1A: in einer schematischen Darstellung eine erste Ausführungsform eines Entnahmebereichs eines Probenentnahmemoduls mit einer gemeinsamen Transportleitung;
- Figur 1B: in einer schematischen Darstellung eine zweite Ausführungsform eines Entnahmebereichs eines Probenentnahmemoduls mit zwei voneinander getrennten Transportleitungen;
- Figur 1C: in einer schematischen Darstellung eine dritte Ausführungsform eines Entnahmebereichs eines Probenentnahmemoduls mit einer teils getrennten und teils gemeinsamen Transportleitung;
- Figur 2: in einer schematischen Darstellung die Funktionsweise der Probenentnahme;
- Figur 3: in einer schematischen Darstellung eine Vorrichtung zur Untersuchung eines Mediums im Inneren eines Einwegbioreaktors;
- Figur 4: in einer schematischen Darstellung eine Vorrichtung zur Untersuchung eines Mediums im Inneren eines Einwegbioreaktors mit einem seriellen Probenentnahmemodul;
- Figur 5: in einer schematischen Darstellung eine Vorrichtung zur Untersuchung eines Mediums im Inneren eines Einwegbioreaktors mit einem parallelen Probenentnahmemodul;
- Figur 6: ein schematisches Ablaufdiagramm zum Ansteuern einer Vorrichtung zur Untersuchung eines Mediums im Inneren eines Einwegbioreaktors mit einem seriellen Probenentnahmemodul; und
- Figur 7: ein schematisches Ablaufdiagramm zum Ansteuern einer Vorrichtung zur Untersuchung eines Mediums im Inneren eines Einwegbioreaktors mit einem parallelen Probenentnahmemodul.

Eine Vorrichtung zur Untersuchung eines Mediums im Inneren eines Bioreaktors weist zumindest ein Probenentnahmemodul auf und kann zudem auch ein Analysemodul aufweisen.

Ein Probenentnahmemodul kann zumindest eine Rohrleitung als Transportleitung aufweisen. Die Rohrleitung kann im Wesentlichen U-förmig, terminiert, fluid transportierend und/oder aus Kunststoff ausgebildet sein. Insbesondere kann die Transportleitung so zweiteilig ausgebildet sein, dass im Inneren der Transportleitung zwei zueinander entgegengesetzte Transportrichtungen bereitgestellt werden, also z.B. eine An- und eine Abtransportleitung.

Das Probenentnahmemodul weist einen Entnahmebereich auf, der als ein medienberührender Abschnitt jeder Rohrleitung ausgebildet sein kann. Der Entnahmebereich ist dazu ausgebildet und vorgesehen, durch die Reaktorwand des Bioreaktors hindurch in das Innere des Bioreaktors so eingeführt zu werden, dass der Entnahmebereich in unmittelbarem Berührkontakt mit dem Medium steht.

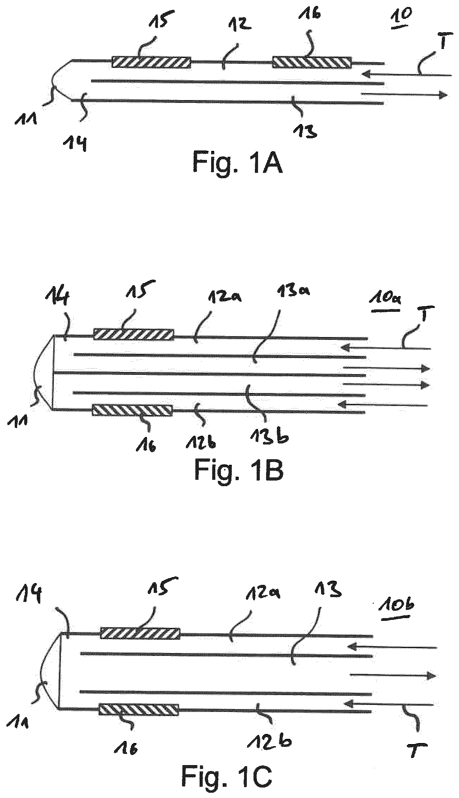

**Figur 1A** zeigt in einer schematischen Darstellung eine erste Ausführungsform eines Entnahmebereichs 10 eines Probenentnahmemoduls mit einer gemeinsamen Transportleitung 14.

Im Entnahmebereich 10 ist zumindest eine Membran angeordnet, insbesondere eine Dialysemembran. Im gezeigten Ausführungsbeispiel weist der Entnahmebereich 10 eine erste Membran 15 und eine zweite Membran 16 auf, welche in Transportrichtung T seriell hintereinander angeordnet sind. Die Membranen 15 und 16 sind jeweils in einer Aussparung in einer Außenwand der Transportleitung 14 so angeordnet, dass sie die Transportleitung 14 an dieser Aussparung zumindest teilweise abdichten. An der jeweiligen Aussparung ersetzt die jeweilige Membran 15, 16 die ausgesparte Leitungswand der Transportleitung 14. Da die Membranen 15 und 16 für vorbestimmte Bestandteile des Mediums zumindest teildurchlässig sind, ist die Abdichtung nicht vollständig sondern eben nur teilweise ausgebildet.

Die Transportrichtung T ist in den Figuren mit Pfeilen gekennzeichnet und folgt dem Verlauf der gemeinsamen Transportleitung 14 durch den Entnahmebereich 10. Die Transportrichtung T verläuft durch einen gemeinsamen Detektionskanal 12 (als Antransportleitung) der Transportleitung 14 bis hin zu einer Sondenspitze 11, an welcher der gemeinsame Detektionskanal 12 terminiert. An der Sondenspitze 11 ist die Transportleitung 14 im Wesentlichen U-förmig ausgebildet, weswegen die Transportrichtung T hier um etwa 180° umgedreht wird. Mit anderen Worten dreht sich die Transportrichtung T an der Sondenspitze 11 um.

Nach dieser Wende an der Sondenspitze 11 verläuft die Transportrichtung T von der Sondenspitze 11 weg entlang eines gemeinsamen Abtransportkanals 13. Der gemeinsame Detektionskanal 12 ist im Wesentlichen parallel zum gemeinsamen Abtransportkanal 13 angeordnet, wobei die Transportrichtungen T durch diese beiden Kanäle 12 und 13 zueinander im Wesentlichen entgegengesetzt sind.

An der Sondenspitze 11 kann der Ein- und Ausgang der Transportleitung 14 mit einem Rohrverbindungselement terminiert sein.

In der Transportleitung 14 werden unterschiedliche Membranen miteinander kombiniert, nämlich die erste Membran 15 und die zweite Membran 16. Diese Membranen 15, 16 können als semipermeable Membranen und/oder Dialysemembranen ausgebildet sein. Die beiden Membranen 15, 16 können unterschiedliche Eigenschaften aufweisen, insbesondere unterschiedliche Cutoff-Porengrößen aufweisen. Deswegen werden durch die beiden Membranen 15, 16 unterschiedliche Bestandteile eines Mediums eines Biorektors zurückzuhalten und/oder durchgelassen. So kann die zweite Membran 16 z.B. eine Cutoff-Porengröße von etwa 10 kDa bis etwa 30 kDa aufweisen, wodurch z.B. nur ein Übertritt von kleineren Molekülen, wie Nährstoffen (z.B. Glucose) und Metaboliten (z.B. Lactat) durch die zweite Membran 16 erfolgen kann. Die erste Membran 15 kann z.B. eine Cutoff-Porengröße von etwa 50 kDa bis etwa 200 kDa aufweisen, wodurch ein Übertritt von etwas größeren Bestandteilen (wie z.B. ein Zielprotein wie ein Antikörper des Mediums) durch die erste Membran 15 erfolgen kann.

Ein Vorteil der zwei hintereinander geschalteten Membranen kann sein, dass in einer ersten Fraktion, welche der ersten Membran zugeordnet ist, eine im Wesentlichen Proteinfreie Probe für z.B. eine Nutrient- und/oder Metabolite-Analyse enthalten sein kann. Diese erste Fraktion kann räumlich und/oder zeitlich von einer zweiten Fraktion sein, welche der zweiten Membran zugeordnet ist. In der zweiten Fraktion können z.B. ein Protein und diverse andere Komponenten wie z.B. Nutrients, Metabolites etc. vorhanden sein. Hierbei kann es vorkommen, evtl. vorhandene Nährstoffe die Analyse und/oder Aufreinigung der Proteine nicht stört. Bei der ersten Fraktion würden für z.B. eine enzymatische Analyse die Proteine und Antikörper durchaus stören. Die erste und zweite Fraktion kann einer ersten und zweiten Probe entsprechen, welche durch die jeweiligen Membrane entnommen werden können.

In einer Ausführungsform kann die zweite Membran als eine 10 kDa Hydrosart Membran ausgebildet sein, z.B. der Sartorius Typ: 14439. In dieser oder einer anderen Ausführungsform kann die erste Membran als eine 100 kDa Hydrosart Membran ausgebildet sein, z.B. der Sartorius Typ: 14468.

Alternativ kann auch ein anderes Material verwendet werden; so dass anstelle des Hydrosarts (einer vernetzten Cellulose) z.B. Polyethersulfon verwendet werden kann, entsprechend den Sartoriustypen 14639 und 14668.

Weiterhin können als Membranen auch
- die Ultracel Reihe (RC Membran von Millipore, Cellulose basiert) und/oder
- die Omega Reihe (PES UF von Pall) und/oder
- Hohlfasern, z.B. aus Polysulfon von Spectrum
   verwendet werden.

Durch die beiden Membranen 15 und 16 können an seriell hintereinander entlang des Transportkanals 14 versetzten Positionen unterschiedliche Bestandteile des Mediums in den denselben, gemeinsamen Transportkanal 14 übertreten. Im Transportkanal 14 kann eine oder mehrere Adsorberlösung(en) als Transportmittel vorgesehen sein. Die Adsorberlösung kann zur Aufnahme der Bestandteile des Mediums in den Transportkanal 14 und zum Transport der aufgenommen Bestandteile entlang des Transportkanals 14 ausgebildet und/oder vorgesehen sein. Die unterschiedlichen Bestandteile des Mediums, die durch die beiden unterschiedlichen Membranen 15, 16 in denselben Transportkanal 14 übertreten, können entlang desselben, gemeinsamen Transportkanals 14 an der Sondenspitze 11 vorbei aus dem Entnahmebereich 10 und dem Bioreaktor hinaus transportiert werden.

**Figur** 1B zeigt in einer schematischen Darstellung eine zweite Ausführungsform eines Entnahmebereichs 10a eines Probenentnahmemoduls. mit zwei voneinander getrennten Transportleitungen 14. Insgesamt ähnelt die zweite Ausführungsform der ersten Ausführungsform, weswegen ähnliche und/oder gleiche Merkmale der Ausführungsformen mit den gleichen Bezugszeichen gekennzeichnet sind.

Im Unterschied zur ersten Ausführungsform weist der in Fig. 1B gezeigte Entnahmebereich 10a zwei voneinander getrennte Transportleitungen 14 auf. Eine erste dieser getrennten Transportleitungen 14 weist einen ersten Detektionskanal 12a auf, welcher an der Sondenspitze 11 umgelenkt ist zu einem ersten Abtransportkanal 13a. Die erste Membran 15 ist in eine Außenwand des ersten Detektionskanals 12a integriert. Erste Bestandteile des Mediums können durch die erste Membran 15 in den ersten Detektionskanal 12a übertreten und durch den ersten Abtransportkanal 13a aus dem Entnahmebereich 10a und aus dem Probenentnahmemodul abtransportiert werden.

Eine zweite der getrennten Transportleitungen 14 weist einen zweiten Detektionskanal 12b auf, welcher an der Sondenspitze 11 umgelenkt ist zu einem zweiten Abtransportkanal 13b. Die zweite Membran 16 ist in eine Außenwand des zweiten Detektionskanals 12b integriert. Zweite Bestandteile des Mediums können durch die zweite Membran 16 in den zweiten Detektionskanal 12b übertreten und durch den zweiten Abtransportkanal 13b separat von den ersten Bestandteilen aus dem Entnahmebereich 10a und aus dem Probenentnahmemodul abtransportiert werden.

**Figur 1C** zeigt in einer schematischen Darstellung eine dritte Ausführungsform eines Entnahmebereichs 10b eines Probenentnahmemoduls mit teils voneinander getrennten Transportleitungen 14 und teilweise gemeinsamen Transportleitungen 14. Insgesamt ähnelt die dritte Ausführungsform der ersten und zweiten Ausführungsform, weswegen ähnliche und/oder gleiche Merkmale der Ausführungsformen mit den gleichen Bezugszeichen gekennzeichnet sind.

Im Unterschied zur zweiten Ausführungsform weist der in Fig. 1C gezeigte Entnahmebereich 10b zwei nur teilweise voneinander getrennte Transportleitungen 14 auf. Ein erster Detektionskanal 12a ist an der Sondenspitze 11 umgelenkt ist zu einem gemeinsamen Abtransportkanal 13. Die erste Membran 15 ist in eine Außenwand des ersten Detektionskanals 12a integriert. Erste Bestandteile des Mediums können durch die erste Membran 15 in den ersten Detektionskanal 12a übertreten und durch den gemeinsamen Abtransportkanal 13 aus dem Entnahmebereich 10b und aus dem Probenentnahmemodul abtransportiert werden.

Ein zweiter Detektionskanal 12b ist an der Sondenspitze 11 ebenfalls umgelenkt zum gemeinsamen Abtransportkanal 13. Die zweite Membran 16 ist in eine Außenwand des zweiten Detektionskanals 12b integriert. Zweite Bestandteile des Mediums können durch die zweite Membran 16 in den zweiten Detektionskanal 12b übertreten und (gemeinsam mit den ersten Bestandteilen) durch den Abtransportkanal 13 aus dem Entnahmebereich 10b und aus dem Probenentnahmemodul abtransportiert werden.

Die in den Figuren 1A, 1B und 1C gezeigten Ausführungsformen können mit umgekehrter Transportrichtung T angesteuert werden.

In den separaten Detektionskanälen 12a und 12b können unterschiedliche Adsorberflüssigkeiten zum Adsorbieren und/oder Transportieren der unterschiedlichen Bestandteile des Mediums geführt sein.

In anderen, nicht in den Figuren gezeigten Ausführungsformen können die Transportleitungen auch anders ausgebildet sein und/oder mehr als zwei unterschiedliche Membrane verwendet werden, z.B. drei Membranen zum Untersuchen zumindest dreier verschiedener Bestandteile des Mediums.

Die Membranen 15 und/oder 16 können mittels unterschiedlicher Fügetechniken an der Transportleitung 14 befestigt sein, welche z.B. aus Kunststoff ausgebildet sein kann. So kann als Technik z.B. "heat sealing" verwendet werden, also ein thermisches Verschweißen der Membran mit dem Kunststoffträger. Z.B. bei einer Cellulose-basierten Membran kann diese Fügetechnik nachteilig sein, da sich dieser Werkstoff nicht so einfach mit einem anderen Kunststoff verschweißen lässt. In diesem Fall, also bei Verwendung einer Cellulose-basierten Membran, können folgende Alternativen verwendet werden:
a) Kleben: Die Membran kann mit einem speziellen Klebstoff verklebt werden. Auf Grund möglicher Probleme mit Extractables/Leachables ist diese Alternative im Biotech Bereich eher ungünstig.
b) Klemmen: Die Membran kann zwischen Kunststoffteile verklemmt und/oder verpresst werden, z.B. auch unter Verwendung von Elastomerdichtungen dazwischen.
c) Overcasten: Hierbei werden Teile der Membran zum Fixieren und Dichten mit einem Duroplasten/Elastomer (z.B. Silikon) überzogen.
d) Ovemolding: Umgießen und/oder Umspritzen der Membran mit einem thermoplastischen Kunststoff.

Diese Fügetechniken können auch bei anderen Membranen verwendet werden.

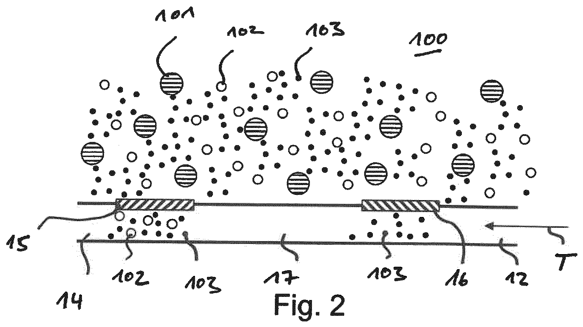

**Figur 2** zeigt in einer schematischen Darstellung die Funktionsweise der Probenentnahme. Hierbei wird insbesondere auf die Probenentnahme mittels eines Probenentnahemoduls eingegangen, dass den in Fig. 1A gezeigten ersten Entnahmebereich 10 mit den seriell hintereinander angeordneten Membranen 15, 16 aufweist. Der erste Entnahmebereich 10 ist lediglich teilweise gezeigt, insbesondere ohne die Sondenspitze 11 und ohne den Abtransportkanal 13.

In der oberen Hälfte der Figur 2 ist ein Medium 100 dargestellt, welches mehrere Bestandteile aufweist. Drei unterschiedliche Bestandteile des Mediums 100 sind durch unterschiedliche Symbole dargestellt. So weist das Medium eine Mehrzahl Zellen 101 auf, welche als quergestreifte große Kreise dargestellt sind. Weiterhin weist das Medium 100 eine Mehrzahl Proteine und/oder Antikörper 102 auf, welche als leere mittelgroße Kreise dargestellt sind. Schließlich weist das Medium 100 eine Mehrzahl kleinere Moleküle 103 auf, welche als kleine Punkte dargestellt sind. Die kleineren Moleküle 103 können z.B. als Glucose und/oder Lactat ausgebildet sein. Die Zellen 101, die Proteine 102 und die kleineren Moleküle 103 sind vermischt miteinander als Medium 100 im Inneren eines nicht gezeigten Bioreaktors angeordnet.

In das Innere des Bioreaktors ist der Entnahmebereich 10 eines Probenentnahmemoduls eingebracht, welcher teilweise in Fig. 2 gezeigt ist. Der Entnahmebereich 10 ist dabei so im Inneren des Bioreaktors angeordnet, dass zumindest die beiden Membranen 15 und 16 in unmittelbarem Berührkontakt mit dem Medium 100 angeordnet sind. Hierbei ist die erste Membran 15 zumindest teilweise als Grenzbereich zwischen dem Medium 100 und dem Inneren des Detektionskanals 12 angeordnet. An einer anderen Stelle der Transportleitung 14 ist die zweite Membran 16 zumindest teilweise als Grenzbereich zwischen dem Medium 100 und dem Inneren des Detektionskanals 12 angeordnet. Zwischen den beiden Membranen 15 und 16 ist im Inneren der Transportleitung 14 ein Zwischenbereich 17 ausgebildet. In Transportrichtung T durch den Detektionskanal 12 ist zuerst die zweite Membran 16 angeordnet, welche an den Zwischenbereich 17 angrenzt, an welchen wiederum die erste Membran 15 angrenzt.

Im gezeigten Ausführungsbeispiel weist die zweite Membran 16 eine kleinere Cutoff-Porengröße auf als die erste Membran 15, z.B. von 10 kDa bis 30 kDa, bevorzugt von 10 kDa bis 20 kDa. Deswegen können durch die zweite Membran 16 nur die kleineren Moleküle 103 in die Transportleitung 14 übertreten und/oder diffundieren. Eine hierzu gewährte zweite Diffundierzeit kann vorbestimmt sein und/oder einstellbar und/oder ansteuerbar und/oder regelbar sein. Während der zweiten Diffundierzeit kann ein in der Transportleitung 14 befindlicher Adsorber nicht angetrieben, sondern ruhen gelassen werden, um den kleineren Molekülen 102 Zeit zu geben, in den Transportkanal 14 überzutreten. Anschließend kann der Adsorber ein Stück weit weiter angetrieben werden, z.B. bis zur ersten Membran 15.

Die erste Membran 15 weist eine größere Cutoff-Porengröße auf als die zweite Membran 16, z.B. von 50 kDa bis 200 kDa, bevorzugt von 75 kDa bis 125 kDa. Deswegen können durch die erste Membran 16 die mittelgroßen Bestandteile des Mediums 100 diffundieren, insbesondere die Proteine und/oder Antikörper 101. Eine hierzu gewährte erste Diffundierzeit kann vorbestimmt sein und/oder einstellbar und/oder ansteuerbar und/oder regelbar sein. Während der ersten Diffundierzeit kann der in der Transportleitung 14 befindliche Adsorber nicht angetrieben, sondern ruhen gelassen werden, um so den mittelgroßen Molekühlen 102 Zeit zu geben, in den Transportkanal 14 überzutreten. Anschließend kann der Adsorber weiter angetrieben werden. Um so sämtliche Proben des Mediums aus dem Probenentnahmemodul heraus zu transportieren und in einem Analysesystem und/oder einem Analysemodul zu analysieren.

Der Adsorber kann über einen externen Antrieb angesteuert werden. Insbesondere kann im Zwischenbereich 17 ein Bereich mit geringer und/oder keiner Aufkonzentration ausgebildet sein, z.B. eine Luftblase als Trennmittel zwischen den beiden Detektionsbereichen im Inneren der Transportleitung 14.

Alternativ kann der Absorber nicht zur ersten und/oder zweiten Diffundierzeit angehalten werden, sondern im Wesentlichen mit konstanter Geschwindigkeit durch die Transportleitung 14 geführt sein.

Durch die schematisch in Fig. 2 gezeigte Probenentnahme können Proben zweier unterschiedlicher Bestandteile des Mediums 100 genommen werden. Diese Proben können in einem externen Analysesystem und/oder einem externen Analysemodul analysiert werden. Beispielsweise kann so die Konzentration der beiden Bestandteile im Medium 100 ermittelt und/oder bestimmt werden.

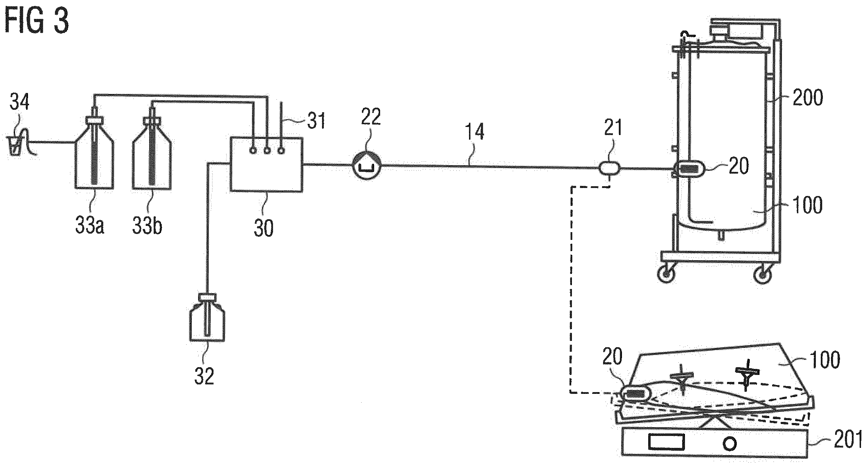

**Figur 3** zeigt in einer schematischen Darstellung eine Vorrichtung zur Untersuchung eines Mediums im Inneren eines Bioreaktors 200 und/oder eines RM-Bioreaktors 201. Der Bioreaktor 200 kann in einem Tank angeordnet sein, z.B. einem Edelstahltank. Der Bioreaktor selber kann als Einwegbioreaktor ausgebildet sein, also z.B. als ein mit dem Medium 100 gefüllter Kunststoffbeutel, welcher in dem gezeigten Tank angeordnet ist. Der Bioreaktor 200 kann z.B. mittels eines Rührmechanismus durchmischt werden. Der Rührmechanismus kann eine angetriebene Achse aufweisen, die in den Bioreaktor 200 hinein ragt. Der Rührmechanismus kann das Medium 100 auch mittels einer rocking-motion (abgekürzt als RM) antreiben, also mittels einer Schaukelbewegung durchmischen. Ein solcher RM-Bioreaktor 201 ist unten in Fig. 3 schematisch gezeigt.

Die Vorrichtung weist ein Probenentnahmemodul 20 auf. Das Probenentnahmemodul 20 weist zumindest einen Entnahmebereich auf, z.B. den in den Figuren 1A, 1B und 1C gezeigten Entnahmebereich 10, 10a und/oder 10b.

Das Probenentnahmemodul 20 ist so angeordnet, dass es die Reaktorwand des Bioreaktors 200 und/oder 201 zumindest teilweise durchdringt. In einer Betriebsposition kann insbesondere der Entnahmebereich 10 des Probenentnahmemoduls 20 vollständig im Inneren des Bioreaktors 200 und/oder 201 angeordnet sein. Hierbei befinden sich insbesondere die beiden Membranen 15 und 16 in unmittelbarem Berührkontakt mit dem Medium 100.

Die Transportleitung 14 durch den Entnahmebereich 10 führt in und aus dem Probenentnahmemodul 20 hinein und hinaus zu einem Analysemodul 30 der Vorrichtung zur Untersuchung des Mediums 100. In dieser extern von dem Probenentnahmemodul 20 angeordneten Transportleitung 14 kann optional eine Sterilverbindung 21 ausgebildet sein. Weiterhin kann zwischen dem Analysemodul 30 und dem Probenentnahmemodul 20 eine Pumpe 22 angeordnet sein, z.B. eine persistaltische Pumpe zum Antreiben eines Inhalts der Transportleitung 14 (also z.B. eines Adsorbers).

Der prinzipielle Aufbau eines Analysesystems zur Messung von Nährstoffen an einem Bioreaktor ist bereits bekannt. Im gezeigten Analysemodul kann jedoch neben den Messungen der Nährstoffe (wie z.B. Glucose, Lactat, etc., also den kleineren Molekülen 103) auch eine Messung eines Gehalts an Zielproteinen 102 durchgeführt werden.

Das Analysemodul 30 kann zumindest eine Messzelle und zumindest einen Analytsensor 31 aufweisen, um die Konzentration zumindest zweier Bestandteile des Mediums 100 zu ermitteln. Bereits untersuchte und/oder nicht benötigte Probenreste könne in einem Abfallbehälter 32 entsorgt werden. Dazu kann das Analysemodul 30 mit dem Abfallbehälter 32 über eine Abfallleitung verbunden sein.

Weiterhin kann das Analysemodul 30 mit einer ersten Kalibrierlösung 33a und einer zweiten Kalibrierlösung 33b verbunden sein, welche zum Kalibrieren des Analysemoduls 30 ausgebildet sind. Eine Überprüfungsvorrichtung 34 kann zum Überprüfen der Kalilbrierlösungen 33a und 33b verwendet werden.

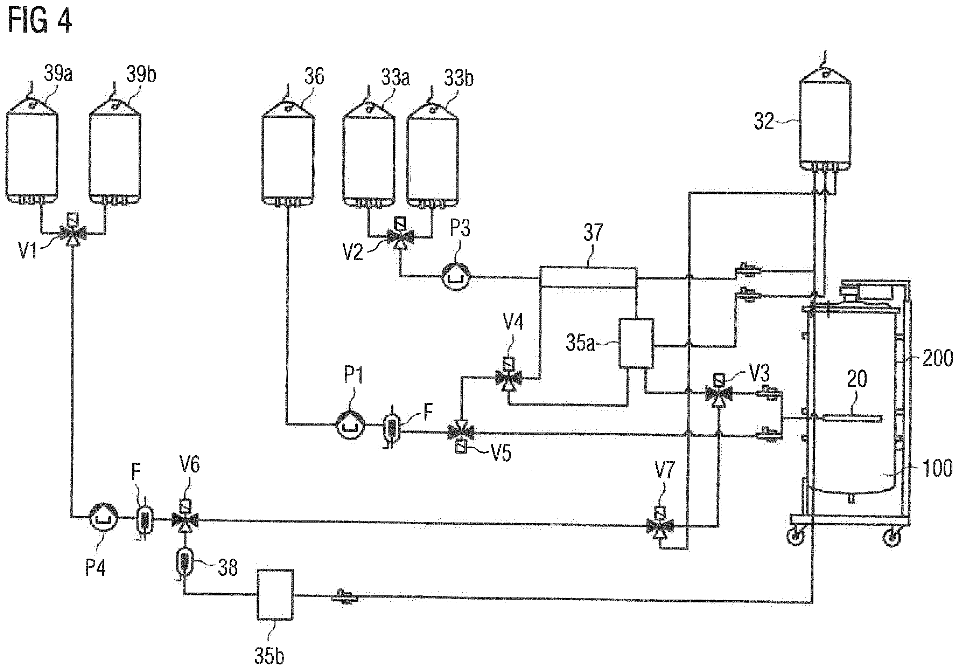

**Figur 4** zeigt in einer schematischen Darstellung eine Vorrichtung zur Untersuchung eines Mediums 100 im Inneren eines Bioreaktors 200 mit einem seriellen Probenentnahmemodul 20 mit mehr Details als Fig. 3. Insbesondere sind in Fig. 4 zusätzliche Bestandteile und/oder Merkmale des Analysemoduls 30 gezeigt. Das serielle Probenentnahmemodul 20 kann z.B. den in Fig. 1A gezeigten Entnahmebereich 10 aufweisen, in welchem die Membranen 15 und 16 seriell hintereinander angeordnet sind.

Eine Transportlösung 36 aus einem Tank dient zum Durchlaufen der Transportleitung 14. Die Transportlösung 36 kann einen Buffer und/oder Adsorber zum Aufnehmen von vorbestimmten Bestandteilen des Mediums 100 enthalten. Die Transportlösung 36 ist über eine erste Messpumpe P1 und ein Filter F mit einem linken Eingang eines fünften Ventils V5 verbunden. Ein rechter Eingang des Ventils V5 ist mit dem Probenentnahmemodul 20 verbunden, z.B. über die Transportleitung 14.

Ein Teilausgang des fünften Ventils V5 ist mit einem linken Eingang eines vierten Ventils V4 verbunden. Ein rechter Eingang des vierten Ventils V4 ist mit einem Diffusionsmodul 37 verbunden. Ein Teilausgang des vierten Ventils V4 ist mit einer ersten Messzelle 35a verbunden. Das Diffusionsmodul 37 ist zudem direkt mit der ersten Messzelle 35a verbunden.

Die erste Messzelle 35a ist mit einem linken Eingang eines dritten Ventils V3 verbunden. Ein rechter Eingang des dritten Ventils V3 ist mit dem Probenentnahmemodul 20 verbunden, z.B. über einen Kanal der Transportleitung 14. Die erste Messzelle 35a ist zudem mit einem Abfallbehälter 32 verbunden, genauso wie das Diffusionsmodul 37.

Eine erste Kalibrierlösung 33a ist mit einem linken Eingang eines zweiten Ventils V2 verbunden. Eine zweite Kalibrierlösung 33b ist mit einem rechten Eingang des zweiten Ventils V2 verbunden. Ein Teilausgang des zweiten Ventils V2 ist über eine dritte Pumpe P3, welche als Samplerpumpe ausgebildet sein kann, mit dem Diffusionsmodul 37 verbunden.

Ein Bindebuffer 39a ist mit einem linken Eingang eines ersten Ventils V1 verbunden. Ein Lösungsbuffer 39b ist mit einem rechten Eingang des ersten Ventils V1 verbunden. Ein Teilausgang des ersten Ventils V1 ist über eine vierte Pumpe P4, welche als Adsorberpumpe ausgebildet sein kann, und über ein Filter F mit einem linken Eingang eines sechsten Ventils V6 verbunden.

Ein rechter Eingang des sechsten Ventils V6 ist mit einem linken Eingang eines siebten Ventils V7 verbunden. Ein Teilausgang des siebten Ventils V7 ist mit dem Abfallbehälter 32 verbunden. Ein rechter Eingang des siebten Ventils V7 ist mit dem Teilausgang des dritten Ventils V3 verbunden.

Ein Teilausgang des sechsten Ventils V6 ist über einen Membranadsorber 38 mit einer zweiten Messzelle 35b verbunden, welche zudem mit dem Abfallbehälter 32 verbunden ist.

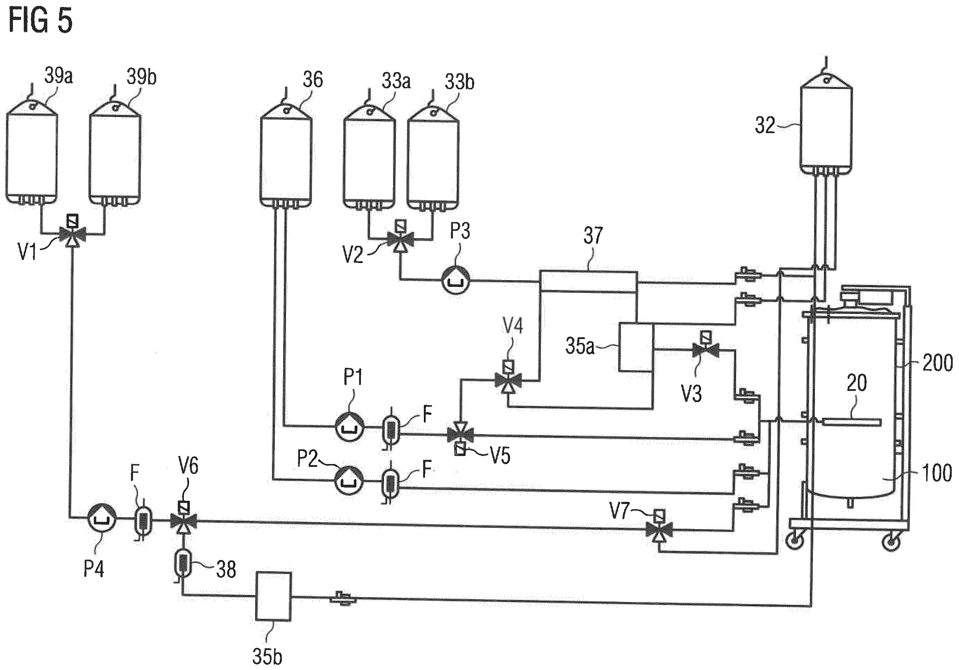

**Figur** 5 zeigt in einer schematischen Darstellung eine Vorrichtung zur Untersuchung eines Mediums 100 im Inneren eines Bioreaktors 200 mit einem parallelen Probenentnahmemodul 20. Das parallele Probenentnahmemodul 20 kann z.B. den in Fig. 1B gezeigten Entnahmebereich 10a aufweisen, in welchem die Membranen 15 und 16 parallel zueinander in zwei voneinander getrennten Transportleitungen 14 angeordnet sind. In Fig. 5 sind zusätzliche Bestandteile und/oder Merkmale des Analysemoduls 30 aus Fig. 3 gezeigt.

Die in Fig. 5 gezeigte Vorrichtung ist in weiten Teilen identisch zu der in Fig. 4 gezeigten Vorrichtung. Im Unterschied zu der in Fig. 4 gezeigten Vorrichtung wird die Transportlösung 36 durch zwei voneinander getrennte Transportleitungen 14 geführt. Deswegen ist der Behälter und/oder Tank mit der Transportlösung 36 zusätzlich über eine zweite Pumpe P2, welche als Messpumpe ausgebildet sein kann, über ein Filter F mit dem Probenentnahmemodul 20 verbunden, genauer gesagt mit der zweiten Transportleitung 14 durch den Entnahmebereich 10a. Hierbei ist die Transportlösung 36 bevorzugt mit dem ersten oder zweiten Detektionskanal 12a oder 12b verbunden. Der zugehörige erste oder zweite Abtransportkanal 13a oder 13b ist mit dem rechten Eingang der siebten Ventils V7 verbunden (und darüber mit der zweiten Messzelle 35b).

Der rechte Eingang des fünften Ventils V5 ist unmittelbar mit dem anderen Detektionskanal 12a oder 12b verbunden und der Teilausgang des dritten Ventils V3 ist geschlossen.

Die Transportlösung 36 kann als Puffer aus dem zugehörigen dem Transportpufferbehälter durch die zweite Transportleitung 14 mit der Membran geleitet werden, die für das Zielprotein 102 durchlässig ist (in den gezeigten Ausführungsbeispielen die erste Membran 15). Die dann mit Protein 102 angereicherte Transportlösung 36 wird durch das siebte Ventil V7 und das sechste Ventil V6 auf den Protein A -Membranadsorber 38 geführt. Eine spezifische Bindung des Protein A zum Fc-Teil der Antikörper kann zu einem selektiven Aufkonzentrieren führen. Nach einer gewissen Zeit, welche einem bestimmten Probevolumen entsprechen kann, wird dann per Ventil umgeschaltet und ein Eluatpuffer (aus dem Lösungspuffer 39b) löst dann den gebundenen Antikörper wieder vom Protein A-Membranadsorber 38. Dieser eluierte Antikörper wird dann in der zweiten Messzelle 35b detektiert. Diese Messzelle 35b kann als eine optische Messzelle ausgebildet sein, die z.B. für Absorptions, Emissions- oder Streumessungen: wie z.B. UVNIS, Fluoreszenz, NIR, MIR, RAMAN, etc. verwendet werden kann. Dabei kann die Detektion z.B. über die Absorption im UV-Bereich bei 280 nm erfolgen. Zusätzlich kann ein eluierter Antikörper zu speziellen Analysen (z.B. biologische automatisierte Assays - Bindungstests, Methoden wie MS, HPLC etc.) zugeführt werden. Anschließend wird der Adsorber mit dem Bindepuffer 39a wieder regeneriert und der Zyklus kann erneut beginnen.

Das für den Protein A-Membranadsorber 38 beschriebene Verfahren kann auch auf andere Modifikationen des Membranadsorbers 38 übertragen werden, dies können andere Proteinmodifikationen sein (z.Bsp. Protein G, Antikörper spezifische Bindungsstrukturen, Rezeptorproteine) oder andere chemische Modifikationen des Membranadsorbers (z.Bsp. Ionenaustauschchromatographie oder hydrophobe Interaktion). Der Verfahrensschritt mit dem Protein A ist optional und kann auch weggelassen werden. In einer Ausführungsform kann dies auch direkt gemessen werden.

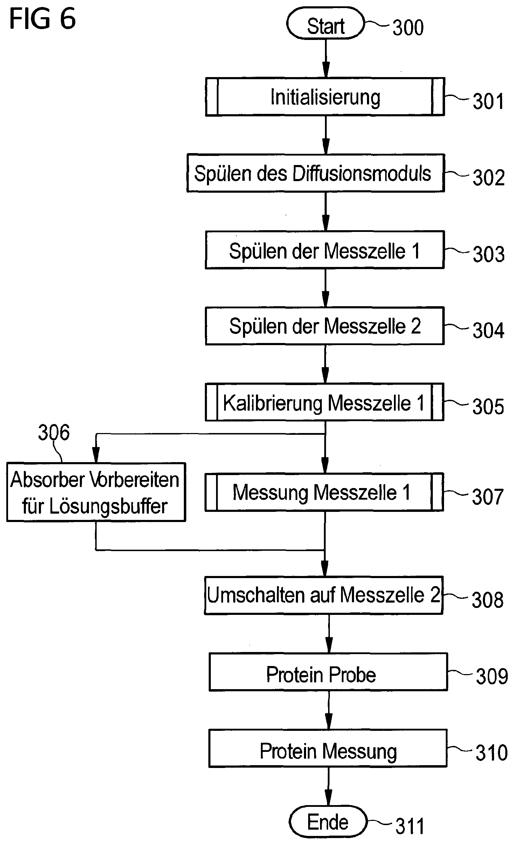

**Figur 6** zeigt ein schematisches Ablaufdiagramm zum Ansteuern einer Vorrichtung zur Untersuchung eines Mediums im Inneren eines Einwegbioreaktors mit einem seriellen Probenentnahmemodul 20, also z.B. der in Fig. 4 gezeigten Vorrichtung.

Zunächst wird das Verfahren im Verfahrensschritt 300 gestartet.

Bei der anschließenden Initialisierung im Verfahrensschritt 301 können die Bestandteile und/oder Module der Vorrichtung auf ihre Startbedingungen gestellt werden und/oder überprüft werden, ob die Bestandteile und/oder Module der Vorrichtung bereits auf ihre jeweilige Startbedingung eingestellt sind. Hierbei können insbesondere die Startbedingungen der Pumpen P1, P3, P4 und/oder Ventile V1 bis V7 eingestellt und/oder überprüft werden.

Bei der in Figur 4 gezeigten Vorrichtung werden im Verfahrensschritt 301 folgende Startbedingungen eingestellt: Das erste, zweite und dritte Ventil V1, V2 und V3 sind geschlossen. Das vierte und siebte Ventil V4 und V7 ist jeweils von Eingang zu Eingang geöffnet. Das fünfte Ventil V5 ist zwischen dem Teilausgang und linkem Eingang geöffnet. Das sechste Ventil V6 ist zwischen dem Teilausgang und dem rechten Eingang geöffnet. Sämtliche Pumpen P1, P3 und P4 sind aus.

Danach wird im Verfahrensschritt 302 das Diffusionsmodul 37 gespült. Dazu wird die erste Messpumpe P1 angeschaltet. Das vierte und fünfte Ventil V4 und V5 sind bereits beim Initialisieren so gestellt worden, dass nun die Transportlösung 36 durch das Diffusionsmodul 37 spült und ggf. vorhandene Reste in den Abfallbehälter 32 spült.

Danach wird im Verfahrensschritt 303 die erste Messzelle 35a gespült. Dazu wird das vierte Ventil V4 zwischen dem Teilausgang und linkem Eingang geöffnet. Dann kann (bei noch immer arbeitender ersten Messpumpe P1) die Transportlösung 36 durch die erste Messzelle 35a gespült werden, wobei wieder ggf. vorhandene Reste in den Abfallbehälter 32 gespült werden.

Danach wird im Verfahrensschritt 304 die zweite Messzelle 35b gespült. Dazu wird das fünfte Ventil V5 von Eingang zu Eingang geöffnet und das dritte Ventil V3 zwischen dem Teilausgang und rechtem Eingang geöffnet. Dann kann (bei noch immer arbeitender ersten Messpumpe P1) die Transportlösung 36 durch die Ventil V5, V7 und V6 hindurch durch die zweite Messzelle 35b gespült werden, wobei wieder ggf. vorhandene Reste in den Abfallbehälter 32 gespült werden.

Danach wird im Verfahrensschritt 305 die erste Messzelle 35a kalibriert. Dazu wird zunächst die erste Messpumpe P1 angeschaltet, das fünfte Ventil V5 vom Teilausgang zum linken Eingang geöffnet, das vierte Ventil V4 von Eingang zu Eingang geöffnet und das zweite Ventil V2 vom Teilausgang zum linken Eingang geöffnet. Eine Kalibriermessung der ersten Kaliberierlösung 33a in der ersten Messzelle 35a wird durchgeführt. Anschließend wird das zweite Ventil V2 geschlossen und die Samplepumpe P3 ausgeschaltet.

Anschließend wird kurze Zeit abgewartet.

Dann wird das zweite Ventil V2 vom Teilausgang zum rechten Eingang geöffnet und die Samplepumpe P3 eingeschalten. Nun kann die erste Kaliberierlösung 33a zum Diffusionsmodul 37 und/oder die erste Messzelle 35a gepumpt werden. Es wird eine weitere Kalibriermessung der ersten Kaliberierlösung 33a in der ersten Messzelle 35a durchgeführt. Anschließend wird das zweite Ventil V2 geschlossen und die Samplepumpe P3 ausgeschaltet.

Die folgenden Verfahrensschritte 307 und 306 können parallel oder alternativ zueinander erfolgen.

Im Verfahrensschritt 306 wird der Adsorber für den Lösungsbuffer 39a vorbereitet. Dazu wird das sechste und erste Ventil V6 und V1 jeweils vom Teilausgang zum linken Eingang geöffnet und die Adsorberpumpe P4 angestellt. Nun wird Lösungsbuffer 39a durch die zweite Messzelle 35b gepumpt.

Im Verfahrensschritt 307 erfolgt eine Messung an der ersten Messzelle 35a. Dazu wird das fünfte Ventil V5 von Eingang zu Eingang geöffnet, das vierte Ventil V4 geschlossen, das dritte Ventil V3 von Eingang zu Eingang geöffnet und die erste Pumpe P1 ausgeschaltet.

Anschließend kann z.B. für einen vorbestimmten Diffusionszeitraum gewartet werden, bis Bestandteile (z.B. die kleineren Moleküle 103) des Mediums 100 als erste Probe durch eine der beiden Membranen 15 bzw. 16 diffundiert sind (in dem in Fig. 2 gezeigten Ausführungsbeispiel durch die zweite Membran 16).

Dann wird die erste Messpumpe P1 angeschaltet bis die so genommene erste Probe in die erste Messzelle 35a transportiert ist. Es erfolgt eine Messung in der ersten Messzelle 35a, insbesondere eine Konzentrationsbestimmung der jeweiligen zu untersuchenden Bestandteile des Mediums 100.

Anschließend wird die erste Messpumpe P1 wieder ausgeschalten.

Nach den beiden Verfahrensschritten 306 und 307 wird im Verfahrensschritt 308 auf die zweite Messzelle 35b umgeschaltet. Dazu wird das dritte Ventil V3 vom Teilausgang zum rechten Eingang geöffnet, so dass Lösungsbuffer 39a durch das sechste und siebte Ventil V6 und V7 in das Probenentnahmemodul 20 gelangt.

Im Verfahrensschritt 309 wird im Probenentnahmemodul eine Probe des Zielproteins 102 genommen. Dazu wird die vierte Pumpe P4 ausgeschaltet, das sechste Ventil V6 vom Teilausgang zum rechten Eingang geöffnet, das erste Ventil V1 geschlossen, und die erste Messpumpe P1 eingeschaltet. Anschließend kann z.B. für einen vorbestimmten Diffusionszeitraum gewartet werden, bis Bestandteile (insbesondere Proteine 102) des Mediums 100 als zweite Probe durch eine der beiden Membranen 15 bzw. 16 diffundiert sind (in dem in Fig. 2 gezeigten Ausführungsbeispiel durch die erste Membran 15).

Im Verfahrensschritt 310 erfolgt eine Protein-Messung an der zweiten Messzelle 35b. Dann wird die erste Messpumpe P1 ausgeschaltet (alternativ kann auch das dritte Ventil V3 von Eingang zu Eingang geöffnet werden für eine weitere Messung an der ersten Messzelle 35a im Verfahrensschritt 307).

Es folgt eine Spülung, wobei das sechste Ventil V6 vom Eingang zu Eingang geöffnet wird, das siebte Ventil V7 vom Teilausgang zum linken Eingang geöffnet wird, das erste Ventil V1 vom Teilausgang zum rechten Eingang geöffnet wird und die vierte Pumpe P4 eingeschaltet wird. Nun verdrängt der Lösungsbuffer 39b den Bindebuffer 39a. Es erfolgt sowohl eine Verdünnung als auch eine Messung in der zweiten Messzelle 35b, wobei das sechste Ventil V6 vom Teilausgang zum linken Eingang geöffnet wird. Der Bindepuffer 39a transportiert den Antikörper zum Membranadsorber und sorgt gleichzeitig für eine gute Bindung des Antikörpers an das Protein des Adsorbers, welche z.B. auch eine klassische Prot. A Säule sein kann. Dies kann auch in mehreren Zyklen geschehen, bis der Adsorber genug beladen ist. Anschließend sorgt der Lösungsbuffer 39b, welcher als Elute-Buffer ausgebildet sein kann, dass der Antikörper wieder vom Protein-A entfernt wird und die hochkonzentrierte Antikörper-Lösung in die Messzelle transportiert wird. Bei der Messung in der zweiten Messzelle 35b kann insbesondere eine Konzentrationsbestimmung der jeweiligen zu untersuchenden Bestandteile des Mediums 100 (hier: des Zielproteins 102) erfolgen.

Mit dem letzten Verfahrensschritt 311 wird das Verfahren beendet. Alternativ kann das Verfahren auch iterativ durchgeführt werden, also statt dessen erneut mit den Verfahrensschritt 300 oder 301 usw. weitergeführt werden.

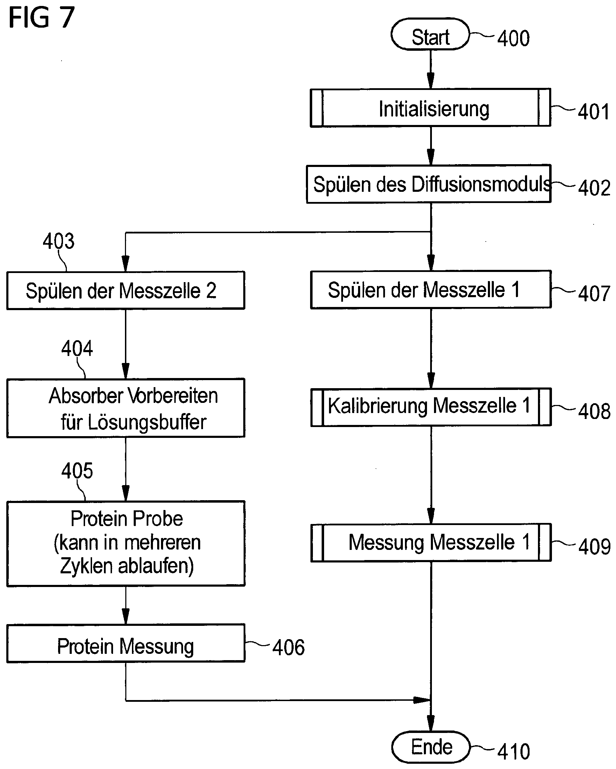

**Figur 7** zeigt ein schematisches Ablaufdiagramm zum Ansteuern einer Vorrichtung zur Untersuchung eines Mediums im Inneren eines Einwegbioreaktors mit einem parallelen Probenentnahmemodul 20, also z.B. der in Fig. 5 gezeigten Vorrichtung.

Zunächst wird das Verfahren im Verfahrensschritt 400 gestartet.

Bei der anschließenden Initialisierung im Verfahrensschritt 401 können die Bestandteile und/oder Module der Vorrichtung auf ihre Startbedingungen gestellt werden und/oder überprüft werden, ob die Bestandteile und/oder Module der Vorrichtung bereits auf ihre jeweilige Startbedingung eingestellt sind. Hierbei können insbesondere die Startbedingungen der Pumpen P1 bis P4 und/oder Ventile V1 bis V7 eingestellt und/oder überprüft werden.

Bei der in Figur 5 gezeigten Vorrichtung werden im Verfahrensschritt 401 folgende Startbedingungen eingestellt: Das erste, zweite, dritte und vierte Ventil V1, V2, V3 und V4 werden geschlossen. Das vierte und siebte Ventil V4 und V7 wird jeweils von Eingang zu Eingang geöffnet. Das fünfte Ventil V5 wird zwischen dem Teilausgang und linkem Eingang geöffnet. Das sechste Ventil V6 wird zwischen dem Teilausgang und dem rechten Eingang geöffnet. Sämtliche Pumpen P1, P3 und P4 sind aus.

Danach wird im Verfahrensschritt 402 das Diffusionsmodul 37 gespült. Dazu wird die erste Messpumpe P1 angeschaltet. Die Transportlösung 36 spült durch das Diffusionsmodul 37 und ggf. vorhandene Reste in den Abfallbehälter 32.

Die folgenden Verfahrensschritte 403 bis 406 betreffen die Messung an der zweiten Messzelle 35b und können parallel oder alternativ zu den Verfahrensschritten 407 bis 409 erfolgen, welche die Messung an der ersten Messzelle 35a betreffen.

So wird im Verfahrensschritt 407 die erste Messzelle 35a gespült. Dazu wird das vierte Ventil V4 zwischen dem Teilausgang und linkem Eingang geöffnet. Dann kann (bei noch immer arbeitender ersten Messpumpe P1) die Transportlösung 36 durch die erste Messzelle 35a gespült werden, wobei wieder ggf. vorhandene Reste in den Abfallbehälter 32 gespült werden.

Im Verfahrensschritt 408 wird die erste Messzelle 35a kalibriert. Dazu wird zunächst die erste Messpumpe P1 angeschaltet, das fünfte Ventil V5 vom Teilausgang zum linken Eingang geöffnet, das vierte Ventil V4 von Eingang zu .Eingang geöffnet und das zweite Ventil V2 vom Teilausgang zum linken Eingang geöffnet. Eine Kalibriermessung der ersten Kaliberierlösung 33a in der ersten Messzelle 35a wird durchgeführt. Anschließend wird das zweite Ventil V2 geschlossen und die Samplepumpe P3 ausgeschalten.

Anschließend wird kurze Zeit abgewartet.

Dann wird das zweite Ventil V2 vom Teilausgang zum rechten Eingang geöffnet und die Samplepumpe P3 eingeschalten. Nun kann die erste Kaliberierlösung 33a zum Diffusionsmodul 37 und/oder die erste Messzelle 35a gepumpt werden. Es wird eine weitere Kalibriermessung der ersten Kaliberierlösung 33a in der ersten Messzelle 35a durchgeführt. Anschließend wird das zweite Ventil V2 geschlossen und die Samplepumpe P3 ausgeschalten.

Im Verfahrensschritt 409 erfolgt eine Messung an der ersten Messzelle 35a. Dazu wird das fünfte Ventil V5 von Eingang zu Eingang geöffnet, das vierte Ventil V4 geschlossen, das dritte Ventil V3 von Eingang zu Eingang geöffnet und eine Messschleife durchgeführt, in welcher:
a) Die erste Pumpe P1 ausgeschaltet wird.
b) Z.B. für einen vorbestimmten Diffusionszeitraum gewartet wird, bis Bestandteile (z.B. die kleineren Moleküle 103) des Mediums 100 als erste Probe durch eine der beiden Membranen 15 bzw. 16 diffundiert sind (in dem in Fig. 2 gezeigten Ausführungsbeispiel durch die zweite Membran 16).
c) Anschließend die erste Messpumpe P1 wieder ausgeschaltet wird.
d) Die erste Messpumpe P1 angeschalten wird, bis die so genommene erste Probe in die erste Messzelle 35a transportiert ist. Es erfolgt eine Messung in der ersten Messzelle 35a, insbesondere eine Konzentrationsbestimmung der jeweiligen zu untersuchenden Bestandteile des Mediums 100.
e) Es wieder kurz abgewartet wird.

Parallel oder alternativ zu diesen Schritten wird im Verfahrensschritt 403 die zweite Messzelle 35b gespült. Dazu wird die zweite Messpumpe P2 angeschaltet, wodurch die Transportlösung 36 durch die Ventil V7 und V6 hindurch durch die zweite Messzelle 35b gespült wird, wobei wieder ggf. vorhandene Reste in den Abfallbehälter 32 gespült werden.

Im Verfahrensschritt 404 wird der Adsorber für den Lösungsbuffer 39a vorbereitet. Dazu wird die zweite Messpumpe ausgeschaltet, das sechste und erste Ventil V6 und V1 jeweils vom Teilausgang zum linken Eingang geöffnet und die Adsorberpumpe P4 angestellt. Nun wird Lösungsbuffer 39a durch die zweite Messzelle 35b gepumpt.

Im Verfahrensschritt 405 wird im Probenentnahmemodul 20 eine Probe des Zielproteins 102 genommen. Dazu wird die vierte Pumpe P4 ausgeschaltet, das sechste Ventil V6 vom Teilausgang zum rechten Eingang geöffnet, das erste Ventil V1 geschlossen, und die zweite Messpumpe P2 eingeschaltet. Anschließend kann z.B. für einen vorbestimmten Diffusionszeitraum gewartet werden, bis Bestandteile (insbesondere Proteine 102) des Mediums 100 als zweite Probe durch eine der beiden Membranen 15 bzw. 16 diffundiert sind (in dem in Fig. 2 gezeigten Ausführungsbeispiel durch die erste Membran 15).

Die zweite Messpumpe P2 wird wieder angeschaltet und es wird auf eine Diffusion in die zweite Messzelle 35 gewartet. Anschließend wird die zweite Messpumpe P2 wieder angeschaltet.

Der Verfahrensschritt 405 kann in mehreren Zyklen ablaufen.

Im Verfahrensschritt 406 erfolgt eine Protein-Messung an der zweiten Messzelle 35b. Zunächst wird die zweite Messpumpe P2 ausgeschaltet.

Es folgt eine Spülung, wobei das sechste Ventil V6 vom Eingang zu Eingang geöffnet wird, das siebte Ventil V7 vom Teilausgang zum linken Eingang geöffnet wird, das erste Ventil V1 vom Teilausgang zum rechten Eingang geöffnet wird und die vierte Pumpe P4 eingeschaltet wird. Nun verdrängt der Lösungsbuffer 39b den Bindebuffer 39a. Es erfolgt sowohl eine Verdünnung als auch eine Messung in der zweiten Messzelle 35b, wobei das sechste Ventil V6 vom Teilausgang zum linken Eingang geöffnet wird. Bei der Messung in der zweiten Messzelle 35b kann insbesondere eine Konzentrationsbestimmung der jeweiligen zu untersuchenden Bestandteile des Mediums 100 (hier: des Zielproteins 102) erfolgen.

Mit dem letzten Verfahrensschritt 410 wird das Verfahren beendet. Alternativ kann das Verfahren auch iterativ durchgeführt werden, also statt dessen erneut mit den Verfahrensschritt 400 oder 301 usw. weitergeführt werden.

Die in den Figuren 6 und 7 grau hinterlegten Verfahrensschritte sind hierbei notwendige Prozessschritte, während einzelne oder sämtliche der weiß hinterlegten Verfahrensschritte weggelassen werden können bzw. nur optional durchgeführt werden.

### Bezugszeichenliste

- 10: Entnahmebereich
- 10a: Entnahmebereich
- 10b: Entnahmebereich
- 11: Probenspitze
- 12: gemeinsamer Detektionskanal
- 12a: erster Detektionskanal
- 12b: zweiter Detektionskanal
- 13: gemeinsamer Abtransportkanal
- 13a: erster Abtransportkanal
- 13b: zweiter Abtransportkanal
- 14: Transportleitung
- 15: erste Membran
- 16: zweite Membran
- 17: Zwischenbereich
- 20: Probenentnahmemodul
- 21: Sterilverbindung
- 22: Pumpe

- 30: Analysemodul
- 31: Analytsensor
- 32: Abfallbehälter
- 33a: erste Kalibrierlösung
- 33b: zweite Kalibrierlösung
- 34: Überprüfungsvorrichtung
- 35a: erste Messzelle
- 35b: zweite Messzelle
- 36: Transportlösung
- 37: Diffusionsmodul
- 38: Membranadsorber
- 39a: Bindebuffer
- 39b: Lösungsbuffer

- 100: Medium
- 101: Zelle
- 102: Protein/Antikörper
- 103: kleines Molekül

- 200: Bioreaktor
- 201: RM Bioreaktor

- F: Filter

- P1: erste Messpumpe
- P2: zweite Messpumpe
- P3: Samplepumpe
- P4: Adsorberpumpe

- V1: erstes Ventil
- V2: zweites Ventil
- V3: drittes Ventil
- V4: viertes Ventil
- V5: fünftes Ventil
- V6: sechstes Ventil
- V7: siebtes Ventil

- T: Transportrichtung

## Patentansprüche

1. Vorrichtung zur Untersuchung eines Mediums (100) im Inneren eines Bioreaktors (200; 201) mit einem Probenentnahmemodul (20) zur Entnahme einer Probe des Mediums (100), wobei
- das Probenentnahmemodul (20) einen Entnahmebereich (10; 10a; 10b) aufweist, der in Berührkontakt mit dem Medium (100) im Inneren des Bioreaktors (200; 201) anordenbar ist;
- am Entnahmebereich (10; 10a; 10b) des Probenentnahmemoduls (20) zumindest zwei unterschiedliche Membranen (15, 16) zur Entnahme einer Probe des Mediums (100) angeordnet sind;
- jede der zumindest zwei Membranen (15, 16) an einem Außenbereich des Entnahmebereichs (10; 10a; 10b) angeordnet ist, so dass jede der zumindest zwei Membranen (15, 16) jeweils in Berührkontakt mit dem Medium (100) im Inneren des Bioreaktors (200; 201) anordenbar ist; und
- die zumindest zwei Membranen (15, 16) sich zumindest dadurch unterscheiden, dass sie unterschiedlich große Cutoff-Porengrößen aufweisen.

2. Vorrichtung nach Anspruch 1, wobei eine erste Membran (15) der zumindest zwei Membranen eine Cuttoff-Porengröße von 50 kDa bis 200 kDa aufweist und eine zweite Membran (16) der zumindest zwei Membranen eine Cuttoff-Porengröße von 10 kDa bis 30 kDa aufweist.

3. Vorrichtung nach einem der vorangegangenen Ansprüche, wobei
- eine erste Membran (15) der zumindest zwei Membranen dazu ausgebildet und angeordnet ist, in Berührkontakt mit dem Medium (100) einen Übertritt von Proteinen (102) aus dem Medium (100) in das Probenentnahmemodul (20) zuzulassen und
- eine zweite Membran (16) der zumindest zwei Membranen dazu ausgebildet und angeordnet ist, in Berührkontakt mit dem Medium (100) einen Übertritt von Nährstoffen (103) und/oder Metaboliten aus dem Medium (100) in das Probenentnahmemodul (20) zuzulassen.

4. Vorrichtung nach einem der vorangegangenen Ansprüche, wobei das Probenentnahmemodul (20) zumindest eine Transportleitung (14) zum Transportieren eines Adsorbers durch den Entnahmebereich (10; 10a; 10b) entlang der Transportleitung (14) aufweist und jede der zumindest zwei Membranen (15, 16) die Transportleitung (14) nach außen begrenzt.

5. Vorrichtung nach Anspruch 4, mit einem Steuermodul zum Steuern eines Adsorberflusses durch die zumindest eine Transportleitung (14) des Probenentnahmemoduls (20).

6. Vorrichtung nach Anspruch 4 oder 5, wobei das Probenentnahmemodul (20) genau eine Transportleitung (14) durch den Entnahmebereich (10; 10a; 10b) aufweist und die zumindest zwei Membranen (15, 16) in Transportrichtung (T) durch die Transportleitung (14) hintereinander angeordnet sind.

7. Vorrichtung nach Anspruch 4 oder 5, wobei das Probenentnahmemodul (20) zumindest zwei Transportleitungen (14) durch den Entnahmebereich (10; 10a; 10b) aufweist und die zumindest zwei Membranen (15, 16) in unterschiedlichen Transportleitungen (14) angeordnet sind.

8. Vorrichtung nach einem der vorangegangenen Ansprüche, wobei die Membranen (15, 16) als Dialysemembranen und/oder semipermeable Membranen ausgebildet sind.

9. Vorrichtung nach einem der vorangegangenen Ansprüche, mit einem Analysemodul (30) zur Untersuchung einer von dem Probenentnahmemodul (20) entnommenen Probe des Mediums (100).

10. Vorrichtung nach Anspruch 9, wobei das Analysemodul (30) dazu ausgebildet ist, durch Untersuchung der entnommenen Probe des Mediums (100) eine Konzentration zumindest zweier verschiedener Bestandteile im Medium (100) zu bestimmen.

11. Verfahren zur Untersuchung eines Mediums (100) im Inneren eines Bioreaktors (200; 201) mit den Schritten:
- Anordnen eines Entnahmebereichs (10; 10a; 10b) eines Probenentnahmemoduls (20) in Berührkontakt mit dem Medium (100) im Inneren des Bioreaktors (200; 201);
- Anordnen jeder von zumindest zwei unterschiedlichen Membranen (15, 16) des Entnahmebereichs (10; 10a; 10b) in Berührkontakt mit dem Medium (100) im Inneren des Bioreaktors (200; 201), wobei jede der zumindest zwei Membranen (15, 16) an einem Außenbereich des Entnahmebereichs (10; 10a; 10b) angeordnet ist;
- Entnehmen einer Probe des Mediums (100) durch die zwei unterschiedlichen Membranen (15, 16) des Entnahmebereichs (10; 10a; 10b) hindurch, wobei sich die zumindest zwei Membranen (15, 16) zumindest dadurch unterscheiden, dass sie unterschiedlich große Cutoff-Porengrößen aufweisen;
- Untersuchen der entnommenen Probe des Mediums (100).

12. Verfahren nach Anspruch 11, wobei die beiden Membranen (15, 16) so in Berührkontakt mit dem Medium (100) angeordnet werden, dass Bestandteile des Mediums (100) durch jede der beiden unterschiedlichen Membranen (15, 16) hindurch in zumindest eine Transportleitung (14) durch den Entnahmebereich (10; 10a; 10b) des Probenentnahmemoduls (20) übertreten.

13. Verfahren nach Anspruch 12, wobei ein Adsorberfluss durch die zumindest eine Transportleitung (14) so angesteuert wird, dass die Bestandteile des Mediums (100) durch jede der Membranen (15, 16) für einen vorbestimmbaren Zeitraum in einen in der Transportleitung (14) ruhenden Adsorber übertreten, bevor der angereichterte Adsorber entlang der zumindest einen Transportleitung (14) zu einem Analysemodul (30) transportiert wird.

14. Verfahren nach einem der Ansprüche 11 bis 13, wobei die Probe des Mediums (100) zumindest zwei Bestandteile aufweist, welche durch eine Luftblase getrennt voneinander durch die beiden unterschiedlichen Membranen (15, 16) hindurch entnommen werden.

## Claims

1. Device for examining a medium (100) in the interior of a bioreactor (200; 201) with a sampling module (20) for taking a sample of the medium (100), wherein
- the sampling module (20) has a sampling region (10; 10a; 10b) which can be arranged in contact with the medium (100) in the interior of the bioreactor (200; 201);
- at the sampling region (10; 10a; 10b) of the sampling module (20) at least two different membranes (15, 16) for taking a sample of the medium (100) are arranged;
- each of the at least two membranes (15, 16) is arranged at an outer region of the sampling region (10; 10a; 10b), such that each of the at least two membranes (15, 16) can in each case be arranged in contact with the medium (100) in the interior of the bioreactor (200; 201); and
- the at least two membranes (15, 16) differ at least in that they have cut-off pore sizes of different size.

2. Device according to claim 1, wherein a first membrane (15) of the at least two membranes has a cut-off pore size of 50 kDa to 200 kDa and a second membrane (16) of the at least two membranes has a cut-off pore size of 10 kDa to 30 kDa.

3. Device according to any of the preceding claims, wherein
- a first membrane (15) of the at least two membranes is designed and arranged to allow, in contact with the medium (100), a passage of proteins (102) from the medium (100) into the sampling module (20) and
- a second membrane (16) of the at least two membranes is designed and arranged to allow, in contact with the medium (100), a passage of nutrients (103) and/or metabolites from the medium (100) into the sampling module (20).

4. Device according to any of the preceding claims, wherein the sampling module (20) has at least one transport line (14) for transporting an adsorber through the sampling region (10; 10a; 10b) along the transport line (14), and each of the at least two membranes (15, 16) delimits the transport line (14) towards the outside.

5. Device according to claim 4, with a control module for controlling an adsorber flow through the at least one transport line (14) of the sampling module (20).

6. Device according to claim 4 or 5, wherein the sampling module (20) has exactly one transport line (14) through the sampling region (10; 10a; 10b) and the at least two membranes (15, 16) are arranged one behind the other in the transport direction (T) through the transport line (14).

7. Device according to claim 4 or 5, wherein the sampling module (20) has at least two transport lines (14) through the sampling region (10; 10a; 10b) and the at least two membranes (15, 16) are arranged in different transport lines (14).

8. Device according to any of the preceding claims, wherein the membranes (15, 16) are designed as dialysis membranes and/or semipermeable membranes.

9. Device according to any of the preceding claims, with an analysis module (30) for examining a sample of the medium (100) removed by the sampling module (20).

10. Device according to claim 9, wherein the analysis module (30) is designed to determine, by examining the removed sample of the medium (100), a concentration of at least two different constituents in the medium (100).

11. Method for examining a medium (100) in the interior of a bioreactor (200; 201), with the steps:
- arranging a sampling region (10; 10a; 10b) of a sampling module (20) in contact with the medium (100) in the interior of the bioreactor (200; 201);
- arranging each of at least two different membranes (15, 16) of the sampling region (10; 10a; 10b) in contact with the medium (100) in the interior of the bioreactor (200; 201), wherein each of the at least two membranes (15, 16) is arranged at an outer region of the sampling region (10; 10a; 10b);
- taking a sample of the medium (100) through the two different membranes (15, 16) of the sampling region (10; 10a; 10b), wherein the at least two membranes (15, 16) differ at least in that they have cut-off pore sizes of different size;
- examining the removed sample of the medium (100).

12. Method according to claim 11, wherein the two membranes (15, 16) are arranged in contact with the medium (100) such that constituents of the medium (100) pass through each of the two different membranes (15, 16) into at least one transport line (14) through the sampling region (10; 10a; 10b) of the sampling module (20).

13. Method according to claim 12, wherein an adsorber flow through the at least one transport line (14) is controlled such that the constituents of the medium (100) pass through each of the membranes (15, 16) for a predeterminable period of time into an adsorber resting in the transport line (14), before the enriched adsorber is transported along the at least one transport line (14) to an analysis module (30).

14. Method according to any of claims 11 to 13, wherein the sample of the medium (100) has at least two constituents which are taken through the two different membranes (15, 16) separated from one another by an air bubble.

## Revendications

1. Dispositif pour l'analyse d'un milieu (100) à l'intérieur d'un bioréacteur (200 ; 201) comprenant un module de prélèvement (20) pour le prélèvement d'un échantillon du milieu (100), dans lequel
- le module de prélèvement (20) présente une zone de prélèvement (10 ; 10a ; 10b) qui est apte à être disposée en contact avec le milieu (100) à l'intérieur du bioréacteur (200 ; 201) ;
- au niveau de la zone de prélèvement (10 ; 10a ; 10b) du module de prélèvement (20) sont disposées au moins deux membranes différentes (15, 16) pour le prélèvement d'un échantillon du milieu (100) ;
- chacune des au moins deux membranes (15, 16) est disposée au niveau d'une zone extérieure de la zone de prélèvement (10 ; 10a ; 10b), de sorte que chacune des au moins deux membranes (15, 16) est respectivement apte à être disposée en contact avec le milieu (100) à l'intérieur du bioréacteur (200 ; 201) ; et
- les au moins deux membranes (15, 16) se distinguent au moins en ce qu'elles présentent des tailles de pores de coupure de tailles différentes.

2. Dispositif selon la revendication 1, dans lequel une première membrane (15) des au moins deux membranes présente une taille de pores de coupure de 50 kDa à 200 kDa et une deuxième membrane (16) des au moins deux membranes présente une taille de pores de coupure de 10 kDa à 30 kDa.

3. Dispositif selon l'une des revendications précédentes, dans lequel
- une première membrane (15) des au moins deux membranes est conçue et disposée pour permettre, en contact avec le milieu (100), un passage de protéines (102) depuis le milieu (100) dans le module de prélèvement (20) et
- une deuxième membrane (16) des au moins deux membranes est conçue et disposée pour permettre, en contact avec le milieu (100), un passage de nutriments (103) et/ou de métabolites depuis le milieu (100) dans le module de prélèvement (20).

4. Dispositif selon l'une des revendications précédentes, dans lequel le module de prélèvement (20) présente au moins une conduite de transport (14) pour le transport d'un adsorbeur à travers la zone de prélèvement (10 ; 10a ; 10b) le long de la conduite de transport (14), et chacune des au moins deux membranes (15, 16) délimite la conduite de transport (14) vers l'extérieur.

5. Dispositif selon la revendication 4, comprenant un module de commande pour la commande d'un flux d'adsorbeur à travers l'au moins une conduite de transport (14) du module de prélèvement (20).

6. Dispositif selon la revendication 4 ou 5, dans lequel le module de prélèvement (20) présente exactement une conduite de transport (14) à travers la zone de prélèvement (10 ; 10a ; 10b) et les au moins deux membranes (15, 16) sont disposées l'une derrière l'autre dans la direction de transport (T) à travers la conduite de transport (14).

7. Dispositif selon la revendication 4 ou 5, dans lequel le module de prélèvement (20) présente au moins deux conduites de transport (14) à travers la zone de prélèvement (10 ; 10a ; 10b) et les au moins deux membranes (15, 16) sont disposées dans des conduites de transport (14) différentes.

8. Dispositif selon l'une des revendications précédentes, dans lequel les membranes (15, 16) sont réalisées sous forme de membranes de dialyse et/ou de membranes semi-perméables.

9. Dispositif selon l'une des revendications précédentes, comprenant un module d'analyse (30) pour l'analyse d'un échantillon du milieu (100) prélevé par le module de prélèvement (20).

10. Dispositif selon la revendication 9, dans lequel le module d'analyse (30) est conçu pour déterminer, par l'analyse de l'échantillon prélevé du milieu (100), une concentration d'au moins deux constituants différents dans le milieu (100).

11. Procédé pour l'analyse d'un milieu (100) à l'intérieur d'un bioréacteur (200 ; 201), comprenant les étapes suivantes :
- agencement d'une zone de prélèvement (10 ; 10a ; 10b) d'un module de prélèvement (20) en contact avec le milieu (100) à l'intérieur du bioréacteur (200 ; 201) ;
- agencement de chacune d'au moins deux membranes différentes (15, 16) de la zone de prélèvement (10 ; 10a ; 10b) en contact avec le milieu (100) à l'intérieur du bioréacteur (200 ; 201), chacune des au moins deux membranes (15, 16) étant disposée au niveau d'une zone extérieure de la zone de prélèvement (10 ; 10a ; 10b) ;
- prélèvement d'un échantillon du milieu (100) à travers les deux membranes différentes (15, 16) de la zone de prélèvement (10 ; 10a ; 10b), les au moins deux membranes (15, 16) se distinguant au moins en ce qu'elles présentent des tailles de pores de coupure de tailles différentes ;
- analyse de l'échantillon prélevé du milieu (100).

12. Procédé selon la revendication 11, dans lequel les deux membranes (15, 16) sont disposées en contact avec le milieu (100) de telle sorte que des constituants du milieu (100) passent à travers chacune des deux membranes différentes (15, 16) dans au moins une conduite de transport (14) à travers la zone de prélèvement (10 ; 10a ; 10b) du module de prélèvement (20).

13. Procédé selon la revendication 12, dans lequel un flux d'adsorbeur à travers l'au moins une conduite de transport (14) est commandé de telle sorte que les constituants du milieu (100) passent à travers chacune des membranes (15, 16), pendant une durée prédéterminable, dans un adsorbeur au repos dans la conduite de transport (14), avant que l'adsorbeur enrichi ne soit transporté le long de l'au moins une conduite de transport (14) vers un module d'analyse (30).

14. Procédé selon l'une des revendications 11 à 13, dans lequel l'échantillon du milieu (100) présente au moins deux constituants qui sont prélevés à travers les deux membranes différentes (15, 16) en étant séparés l'un de l'autre par une bulle d'air.
